(19) Europäisches Patentamt
European Patent Office
Office européen des brevets

(11) EP 4 166 144 A1

(12) # EUROPEAN PATENT APPLICATION
published in accordance with Art. 153(4) EPC

(43) Date of publication:
**19.04.2023 Bulletin 2023/16**

(21) Application number: **21823093.6**

(22) Date of filing: **07.06.2021**

(51) International Patent Classification (IPC):
***A61K 31/713*** (2006.01) ***A61P 31/20*** (2006.01)
***C12N 15/113*** (2010.01)

(52) Cooperative Patent Classification (CPC):
**A61K 31/713; A61P 31/20; C12N 15/113**

(86) International application number:
**PCT/CN2021/098682**

(87) International publication number:
**WO 2021/249352 (16.12.2021 Gazette 2021/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.06.2020 CN 202010529520**
**21.12.2020 CN 202011524835**

(71) Applicants:
- **CHIA TAI TIANQING PHARMACEUTICAL GROUP CO., LTD.**
  **Lianyungang,**
  **Jiangsu 222062 (CN)**
- **Medshine Discovery Inc.**
  **Nanjing, Jiangsu 210032 (CN)**

(72) Inventors:
- **AN, Ke**
  **Shanghai 200131 (CN)**
- **SUN, Fei**
  **Shanghai 200131 (CN)**
- **DING, Charles Z.**
  **Shanghai 200131 (CN)**
- **CHEN, Shuhui**
  **Shanghai 200131 (CN)**

(74) Representative: **Gulde & Partner**
**Patent- und Rechtsanwaltskanzlei mbB**
**Wallstraße 58/59**
**10179 Berlin (DE)**

Remarks:
The complete document including Reference Table(s) and the Sequence Listing(s) can be downloaded from the EPO website

(54) **CONJUGATE OF DOUBLE-STRANDED SIRNA ANALOGUE**

(57) Provided are a double-stranded siRNA analogue embedded with a ribavirin derivative, a conjugate containing same, and a salt and the use thereof. The provided double-stranded siRNA analogue, the conjugate containing same and the salt thereof can effectively inhibit multiple viral indicators such as hepatitis B virus DNA, pgRNA, S antigen, and E antigen, which provide an effective and feasible method for treating hepatitis B.

EP 4 166 144 A1

## Description

The present application claims priority to:

CN202010529520.7 filed on Jun. 11, 2020;
CN202011524835.9 filed on Dec. 21, 2020;
CN202010524584.8 filed on Jun. 10, 2020;
PCT/CN2020/133982 filed on Dec. 4, 2020;
CN202010522407.6 filed on Jun. 10, 2020; and
CN202011524307.3 filed on Dec. 21, 2020.

## TECHNICAL FIELD

The present disclosure belongs to the field of biomedicine and relates to an r'-embedded siRNA analogue, a double-stranded siRNA analogue, a conjugate containing the same, and a salt and use thereof; the use is specifically use for preparing a medicament for the treatment of viral hepatitis B.

## BACKGROUND

Viral hepatitis B, abbreviated as hepatitis B, is a disease caused by hepatitis B virus (HBV) infection of the body. Hepatitis B virus is a hepatotropic virus, and it is mainly present in and damages hepatocytes, causing inflammation, necrosis and fibrosis of hepatocytes. Viral hepatitis B is classified into acute and chronic ones. Acute hepatitis B can be mostly self-healed in adults by their own immune mechanisms. However, chronic hepatitis B (CHB) has become a great challenge for global health care and is also the main cause of chronic liver disease, cirrhosis and hepatic carcinoma (HCC) (Edward J. G., et al., The oral toll-like receptor-7 agonist GS-9620 in patients with chronic hepatitis B virus infection. Journal of Hepatology (2015); 63: 320-328). It is estimated that 2 billion people worldwide have been infected with CHB virus, over 350 million of whom have developed hepatitis B, and nearly 600,000 people die annually from complications of CHB (Edward J. G., et al., The oral toll-like receptor 7 agonist GS-9620 in patients with chronic hepatitis B virus infection. Journal of Hepatology (2015)). China is a high-incidence area of hepatitis B and has a large number of hepatitis B patients, which causes great harm. The data show that now there are about 93 million patients infected with hepatitis B virus in China, and about 20 million of the patients are diagnosed with chronic hepatitis B, 10-20% of whom may develop cirrhosis and 1-5% of whom may develop hepatic carcinoma. (Zhang Chunhong, Application of interferon in the treatment of hepatitis B. Guide of China Medicine (2013); 11: 475-476).

The key to the functional cure of hepatitis B is the clearance of HBsAg (hepatitis B surface antigen) and the production of surface antibodies. HBsAg quantification is a very important biological indicator. In chronically infected patients, reduction in HBsAg and seroconversion are rarely observed, which is the endpoint of current therapy.

Currently approved anti-HBV drugs on the market are mainly immunomodulators (interferon-$\alpha$ and polyethylene glycol interferon-$\alpha$-2$\alpha$) and antiviral therapeutic drugs (lamivudine, adefovir dipivoxil, entecavir, telbivudine, tenofovir, clevudine, etc.). Among them, the antiviral therapeutic drugs belong to the nucleotide drugs, and the action mechanism thereof is to inhibit the synthesis of HBV DNA, but the HBsAg level cannot be directly reduced. As with the extended therapy, the nucleotide drugs show HBsAg clearance rates similar to those observed naturally (Janssen et al., Lancet (2005), 365, 123-129; Marcellin et al., N. Engl.J.Med. (2004), 351, 1206-1217; Buster et al., Hepatology (2007), 46, 388-394).

There have been therapies for reducing HBsAg in clinic, but the curative effect is poor. Therefore, if the gene expression of the virus can be silenced from the gene level to block the generation and replication of HBV, especially the production of HBsAg and HBeAg (hepatitis B S antigen and E antigen), the virus metabolism and the infection of liver cells by the virus can be fundamentally reduced. Small interfering RNA (siRNA) can, based on the RNA interference (RNAi) mechanism, inhibit or block the expression of a target gene in a sequence-specific manner and perform an inhibitory effect in mRNA translation to protein, thereby achieving the purpose of treating diseases (WO2016077321, WO2018195165). With respect to this most ideal therapeutic means for hepatitis B, stabilized modification of siRNA and auxiliary corresponding delivery systems for target organs and cells are needed to improve metabolic stability, but the current siRNA cannot effectively reduce the content of hepatitis B virus S antigen and E antigen.

Meanwhile, the siRNA can, through partial complementary pairing with certain mRNA fragments, play a role in regulating the expression of a gene corresponding to the mRNA. In particular, the complementary pairing of the seed region at the 5' end of the antisense strand of the siRNA with a non-targeted gene partially or completely silences the gene expression, and this phenomenon is the main cause of off-target effect of siRNA *in vivo* and *in vitro* (Jackson et al., RNA (2006), 12, 1179-1187). siRNAs for treating hepatitis B showed this drawback both in the clinical and preclinical stages (WO2020036862). Although the risk of off-target can be reduced by some modifications of the nucleotides (Iribe

et al., ACS Omega (2017), 2, 2055-2064; Janas et al., Nat. Commun. 2018, 9, 723-732), the effectiveness of silencing is also reduced and the therapeutic safety window remains to be improved.

## SUMMARY

The present disclosure relates to a double-stranded siRNA analogue embedded with a ribavirin derivative, a conjugate comprising same, and a salt and use thereof. The double-stranded siRNA analogue, the conjugate comprising the same and the salt thereof of the present disclosure can effectively inhibit a plurality of virus indicators, such as hepatitis B virus DNA, S antigen and E antigen, and provide an effective and feasible means for treating (e.g., functionally curing) hepatitis B, such as chronic hepatitis B.

Therefore, in the first aspect, the present disclosure provides a double-stranded siRNA analogue comprising a sense strand and an antisense strand, wherein the antisense strand comprises a sequence obtained by replacing one or more nucleotide residues in a sequence set forth in SEQ ID NO: 2 with r, and the r is

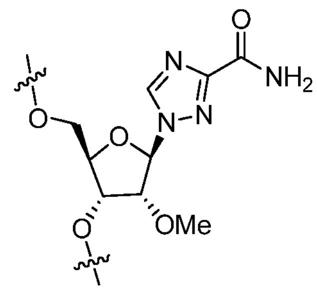

**r**

,

wherein each of nucleotides and r in the siRNA analogue is independently modified or unmodified. In some embodiments, one or more of the nucleotides and r in the siRNA analogue are modified, while the other nucleotides and r are unmodified. The modification includes, for example, methoxy modification, fluoro modification, phosphorothioate linkage, replacement of a nucleotide with (*S*)-glycerol nucleic acid or the like.

In some embodiments, one or more of the nucleotides and r in the siRNA analogue are modified, while the other nucleotides and r are unmodified. The modification includes, for example, methoxy modification, fluoro modification, phosphorothioate linkage, replacement of a nucleotide with (*S*)-glycerol nucleic acid, replacement of a nucleotide with (*E*)-vinyl phosphate or the like.

In some embodiments, substantially all of the nucleotides and r in the siRNA analogue are modified. In some embodiments, all of the nucleotides and r in the siRNA analogue are modified. In some embodiments, 70%, 75%, 80%, 85%, 90%, or 95% or more of the nucleotides and r in the double-stranded siRNA analogue are modified. In some embodiments, all of the nucleotides and r in the double-stranded siRNA analogue are modified.

In some embodiments, the SEQ ID NO: 2 optionally comprises an overhang at the 5' end and/or 3' end. In some embodiments, the SEQ ID NO: 2 comprises an overhang of 0, 1, 2, 3, 4 or 5 nucleotides at the 5' end and/or 3' end.

In some embodiments, when the SEQ ID NO: 2 comprises an overhang of 2 nucleotides at the 5' end and/or 3' end, there are optionally 2 phosphorothioate linkages between the 3 nucleotides at the end, wherein 2 of the 3 nucleotides are the overhang and the other 1 nucleotide is the pairing nucleotide adjacent to the overhang. In some embodiments, the overhang is preferably selected from modified or unmodified UU. In some embodiments, the overhang is preferably selected from uu. In some embodiments, there are 2 phosphorothioate linkages between the overhang uu and 1 pairing nucleotide adjacent thereto.

In some embodiments, the SEQ ID NO: 2 comprises an overhang at the 3' end, and the overhang is preferably selected from modified or unmodified UU. In some embodiments, the SEQ ID NO: 2 comprises an overhang at the 3' end, and the overhang is preferably selected from uu. In some embodiments, the SEQ ID NO: 2 comprises an overhang at the 3' end, and there are 2 phosphorothioate linkages between the overhang uu and 1 pairing nucleotide adjacent thereto (e.g., c•u•u).

In some embodiments, the antisense strand in the double-stranded siRNA analogue comprises a sequence obtained by replacing one or more nucleotide residues in a sequence set forth in SEQ ID NO: 2 with r. For example, the antisense strand comprises a sequence obtained by replacing one nucleotide residue in a sequence set forth in SEQ ID NO: 2 with r.

In some embodiments, the antisense strand in the double-stranded siRNA analogue comprises a sequence obtained by replacing one or more nucleotide residues in a sequence set forth in SEQ ID NO: 2 with r. For example, the antisense strand comprises a sequence obtained by replacing one, two, three, four or five nucleotide residues in the

sequence set forth in SEQ ID NO: 2 with r.

In some embodiments, the antisense strand in the double-stranded siRNA analogue comprises a sequence obtained by replacing one or more nucleotide residues in a sequence set forth in SEQ ID NO: 2 with r, and the r replacement occurs at any position of the SEQ ID NO: 2. Preferably, the r replacement occurs at positions 1 to 21 or 1 to 19 of the 5' end of SEQ ID NO: 2. For example, the r replacement occurs at position 1, 2, 3, 4, 5, 6, 7, 8, 9, 10, 11, 12, 13, 14, 15, 16, 17, 18, 19, 20, or 21 of the 5' end of SEQ ID NO: 2. Preferably, the r replacement occurs at position 2, 3, 4, 5, 6, 7, 8, 10, 11, 12, 16 or 18 of the 5' end of SEQ ID NO: 2.

In some embodiments, the antisense strand in the double-stranded siRNA analogue comprises or consists of a sequence set forth in SEQ ID NO: 4 or SEQ ID NO: 17, SEQ ID NO: 6 or SEQ ID NO: 19, SEQ ID NO: 7 or SEQ ID NO: 20, SEQ ID NO: 8 or SEQ ID NO: 21, SEQ ID NO: 9 or SEQ ID NO: 22, SEQ ID NO: 10 or SEQ ID NO: 23, SEQ ID NO: 11 or SEQ ID NO: 24, SEQ ID NO: 29 or SEQ ID NO: 33, SEQ ID NO: 30 or SEQ ID NO: 34, SEQ ID NO: 31 or SEQ ID NO: 35, or SEQ ID NO: 32 or SEQ ID NO: 36. In some embodiments, the sequence comprises further nucleotide modifications, such as methoxy modification, fluoro modification, phosphorothioate linkage, or replacement of a nucleotide with (*S*)-glycerol nucleic acid or the like.

In some embodiments, the antisense strand in the double-stranded siRNA analogue comprises or consists of a sequence set forth in SEQ ID NO: 4 or SEQ ID NO: 17, SEQ ID NO: 6 or SEQ ID NO: 19, SEQ ID NO: 7 or SEQ ID NO: 20, SEQ ID NO: 8 or SEQ ID NO: 21, SEQ ID NO: 9 or SEQ ID NO: 22, SEQ ID NO: 10 or SEQ ID NO: 23, SEQ ID NO: 11 or SEQ ID NO: 24, SEQ ID NO: 29 or SEQ ID NO: 33, SEQ ID NO: 30 or SEQ ID NO: 34, SEQ ID NO: 31 or SEQ ID NO: 35, SEQ ID NO: 32 or SEQ ID NO: 36, SEQ ID NO: 39 or SEQ ID NO: 44, SEQ ID NO: 10 or SEQ ID NO: 45, SEQ ID NO: 40 or SEQ ID NO: 46, SEQ ID NO: 10 or SEQ ID NO: 47, or SEQ ID NO: 10 or SEQ ID NO: 48. In some embodiments, the sequence comprises further nucleotide modifications, such as methoxy modification, fluoro modification, phosphorothioate linkage, replacement of a nucleotide with (*S*)-glycerol nucleic acid, or replacement of a nucleotide with (*E*)-vinyl phosphate or the like.

In some embodiments, the sense strand in the double-stranded siRNA analogue comprises or consists of a sequence set forth in SEQ ID NO: 1 or SEQ ID NO: 28.

In some embodiments, the sense strand in the double-stranded siRNA analogue comprises a sequence obtained by replacing one or more nucleotide residues in a sequence set forth in SEQ ID NO: 1 with r. For example, the sense strand comprises a sequence obtained by replacing one nucleotide residue in a sequence set forth in SEQ ID NO: 1 with r.

In some embodiments, the sense strand in the double-stranded siRNA analogue comprises a sequence obtained by replacing one or more nucleotide residues in a sequence set forth in SEQ ID NO: 1 with r. For example, the sense strand comprises a sequence obtained by replacing 1, 2, 3, 4 or 5 nucleotide residues in a sequence set forth in SEQ ID NO: 1 with r.

In some embodiments, the sense strand in the double-stranded siRNA analogue comprises a sequence obtained by replacing one or more nucleotide residues in a sequence set forth in SEQ ID NO: 1 with r, and the r replacement occurs at positions 1 to 19 of the 5' end of SEQ ID NO: 1. For example, the r replacement occurs at position 1, 2, 3, 4, 5, 6, 7, 8, 9, 10, 11, 12, 13, 14, 15, 16, 17, 18 or 19 of the 5' end of SEQ ID NO: 1. Preferably, the r replacement occurs at position 2, 3, 7, 12, 15, 17 or 19 of the 5' end of SEQ ID NO: 1.

In some embodiments, the sequence of the sense strand in the double-stranded siRNA analogue comprises or consists of a sequence set forth in SEQ ID NO: 5 or SEQ ID NO: 18, SEQ ID NO: 3 or SEQ ID NO: 16, SEQ ID NO: 14 or SEQ ID NO: 27, SEQ ID NO: 13 or SEQ ID NO: 26, or SEQ ID NO: 12 or SEQ ID NO: 25. In some embodiments, the sequence comprises further nucleotide modifications, such as methoxy modification, fluoro modification or phosphorothioate linkage or the like.

In some embodiments, the sequence of the sense strand in the double-stranded siRNA analogue comprises or consists of a sequence set forth in SEQ ID NO: 5 or SEQ ID NO: 18, SEQ ID NO: 3 or SEQ ID NO: 16, SEQ ID NO: 14 or SEQ ID NO: 27, SEQ ID NO: 13 or SEQ ID NO: 26, SEQ ID NO: 12 or SEQ ID NO: 25, SEQ ID NO: 37 or SEQ ID NO: 42, or SEQ ID NO: 38 or SEQ ID NO: 43. In some embodiments, the sequence comprises further nucleotide modifications, such as methoxy modification, fluoro modification, phosphorothioate linkage, replacement of a nucleotide with (*S*)-glycerol nucleic acid, or replacement of a nucleotide with (*E*)-vinyl phosphate or the like. In some specific embodiments, the sense strand and antisense strand of the double-stranded siRNA analogue comprise a sequence obtained by replacing one or more nucleotide residues in a sequence with r, such as the sequence set forth in the antisense strand SEQ ID NO: 2 (the r replacement occurring at position 2 of the 5' end of SEQ ID NO: 2) and the sequence set forth in the sense strand SEQ ID NO: 1 (the r replacement occurring at position 7 of the 5' end).

In some embodiments, the double-stranded siRNA analogue is any one of S18-S28:

S18: the sense strand is SEQ ID NO: 1 or SEQ ID NO: 28, and the antisense strand is SEQ ID NO: 4 or SEQ ID NO: 17,
S19: the sense strand is SEQ ID NO: 1 or SEQ ID NO: 28, and the antisense strand is SEQ ID NO: 6 or SEQ ID NO: 19,
S20: the sense strand is SEQ ID NO: 1 or SEQ ID NO: 28, and the antisense strand is SEQ ID NO: 7 or SEQ ID NO: 20,
S21: the sense strand is SEQ ID NO: 1 or SEQ ID NO: 28, and the antisense strand is SEQ ID NO: 8 or SEQ ID NO: 21,

S22: the sense strand is SEQ ID NO: 1 or SEQ ID NO: 28, and the antisense strand is SEQ ID NO: 9 or SEQ ID NO: 22,
S23: the sense strand is SEQ ID NO: 1 or SEQ ID NO: 28, and the antisense strand is SEQ ID NO: 10 or SEQ ID NO: 23,
S24: the sense strand is SEQ ID NO: 1 or SEQ ID NO: 28, and the antisense strand is SEQ ID NO: 11 or SEQ ID NO: 24,
S25: the sense strand is SEQ ID NO: 1 or SEQ ID NO: 28, and the antisense strand is SEQ ID NO: 29 or SEQ ID NO: 33,
S26: the sense strand is SEQ ID NO: 1 or SEQ ID NO: 28, and the antisense strand is SEQ ID NO: 30 or SEQ ID NO: 34,
S27: the sense strand is SEQ ID NO: 1 or SEQ ID NO: 28, and the antisense strand is SEQ ID NO: 31 or SEQ ID NO: 35, and
S28: the sense strand is SEQ ID NO: 1 or SEQ ID NO: 28, and the antisense strand is SEQ ID NO: 32 or SEQ ID NO: 36.

In some embodiments, the double-stranded siRNA analogue is any one of S1-S17:

S1: the sense strand is SEQ ID NO: 3 or SEQ ID NO: 16, and the antisense strand is SEQ ID NO: 4 or SEQ ID NO: 17,
S2: the sense strand is SEQ ID NO: 5 or SEQ ID NO: 18, and the antisense strand is SEQ ID NO: 4 or SEQ ID NO: 17,
S3: the sense strand is SEQ ID NO: 3 or SEQ ID NO: 16, and the antisense strand is SEQ ID NO: 6 or SEQ ID NO: 19,
S4: the sense strand is SEQ ID NO: 5 or SEQ ID NO: 18, and the antisense strand is SEQ ID NO: 6 or SEQ ID NO: 19,
S5: the sense strand is SEQ ID NO: 3 or SEQ ID NO: 16, and the antisense strand is SEQ ID NO: 7 or SEQ ID NO: 20,
S6: the sense strand is SEQ ID NO: 5 or SEQ ID NO: 18, and the antisense strand is SEQ ID NO: 7 or SEQ ID NO: 20,
S7: the sense strand is SEQ ID NO: 3 or SEQ ID NO: 16, and the antisense strand is SEQ ID NO: 8 or SEQ ID NO: 21,
S8: the sense strand is SEQ ID NO: 5 or SEQ ID NO: 18, and the antisense strand is SEQ ID NO: 8 or SEQ ID NO: 21,
S9: the sense strand is SEQ ID NO: 3 or SEQ ID NO: 16, and the antisense strand is SEQ ID NO: 9 or SEQ ID NO: 22,
S10: the sense strand is SEQ ID NO: 5 or SEQ ID NO: 18, and the antisense strand is SEQ ID NO: 9 or SEQ ID NO: 22,
S11: the sense strand is SEQ ID NO: 3 or SEQ ID NO: 16, and the antisense strand is SEQ ID NO: 10 or SEQ ID NO: 23,
S12: the sense strand is SEQ ID NO: 5 or SEQ ID NO: 18, and the antisense strand is SEQ ID NO: 10 or SEQ ID NO: 23,
S13: the sense strand is SEQ ID NO: 3 or SEQ ID NO: 16, and the antisense strand is SEQ ID NO: 11 or SEQ ID NO: 24,
S14: the sense strand is SEQ ID NO: 5 or SEQ ID NO: 18, and the antisense strand is SEQ ID NO: 11 or SEQ ID NO: 24,
S15: the sense strand is SEQ ID NO: 12 or SEQ ID NO: 25, and the antisense strand is SEQ ID NO: 4 or SEQ ID NO: 17,
S16: the sense strand is SEQ ID NO: 13 or SEQ ID NO: 26, and the antisense strand is SEQ ID NO: 4 or SEQ ID NO: 17, and
S17: the sense strand is SEQ ID NO: 14 or SEQ ID NO: 27, and the antisense strand is SEQ ID NO: 4 or SEQ ID NO: 17.

In some embodiments, the double-stranded siRNA analogue is any one of S29-S35:

S29: the sense strand is SEQ ID NO: 37 or SEQ ID NO: 42, and the antisense strand is SEQ ID NO: 10 or SEQ ID NO: 23,
S30: the sense strand is SEQ ID NO: 38 or SEQ ID NO: 43, and the antisense strand is SEQ ID NO: 10 or SEQ ID NO: 23,
S31: the sense strand is SEQ ID NO: 3 or SEQ ID NO: 16, and the antisense strand is SEQ ID NO: 39 or SEQ ID NO: 44,
S32: the sense strand is SEQ ID NO: 3 or SEQ ID NO: 16, and the antisense strand is SEQ ID NO: 10 or SEQ ID NO: 45,
S33: the sense strand is SEQ ID NO: 3 or SEQ ID NO: 16, and the antisense strand is SEQ ID NO: 40 or SEQ ID NO: 46,
S34: the sense strand is SEQ ID NO: 3 or SEQ ID NO: 16, and the antisense strand is SEQ ID NO: 10 or SEQ ID NO: 47, and
S35: the sense strand is SEQ ID NO: 3 or SEQ ID NO: 16, and the antisense strand is SEQ ID NO: 10 or SEQ ID NO: 48.

In some embodiments, the double-stranded siRNA analogue is selected from the following: the sense strand is SEQ ID NO: 3 and the antisense strand is SEQ ID NO: 4, the sense strand is SEQ ID NO: 5 and the antisense strand is SEQ ID NO: 4, the sense strand is SEQ ID NO: 3 and the antisense strand is SEQ ID NO: 6, the sense strand is SEQ ID NO: 5 and the antisense strand is SEQ ID NO: 6, the sense strand is SEQ ID NO: 3 and the antisense strand is SEQ ID NO: 7, the sense strand is SEQ ID NO: 5 and the antisense strand is SEQ ID NO: 7, the sense strand is SEQ ID NO: 3 and the antisense strand is SEQ ID NO: 8, the sense strand is SEQ ID NO: 5 and the antisense strand is SEQ ID NO: 8, the sense strand is SEQ ID NO: 3 and the antisense strand is SEQ ID NO: 9, the sense strand is SEQ ID NO: 5 and the antisense strand is SEQ ID NO: 9, the sense strand is SEQ ID NO: 3 and the antisense strand is SEQ ID NO: 10, the sense strand is SEQ ID NO: 5 and the antisense strand is SEQ ID NO: 10, the sense strand is SEQ ID NO: 3 and the antisense strand is SEQ ID NO: 11, the sense strand is SEQ ID NO: 5 and the antisense strand is SEQ ID NO: 11, the sense strand is SEQ ID NO: 12 and the antisense strand is SEQ ID NO: 4, the sense strand is SEQ ID NO: 13 and the antisense strand is SEQ ID NO: 4, the sense strand is SEQ ID NO: 14 and the antisense strand is SEQ ID NO: 4, the sense strand is SEQ ID NO: 1 and the antisense strand is SEQ ID NO: 4, the sense strand is SEQ ID NO: 1 and the antisense strand is SEQ ID NO: 6, the sense strand is SEQ ID NO: 1 and the antisense strand is SEQ ID NO: 7, the sense strand is SEQ ID NO: 1 and the antisense strand is SEQ ID NO: 8, the sense strand is SEQ ID NO: 1 and the

antisense strand is SEQ ID NO: 9, the sense strand is SEQ ID NO: 1 and the antisense strand is SEQ ID NO: 10, the sense strand is SEQ ID NO: 1 and the antisense strand is SEQ ID NO: 11, the sense strand is SEQ ID NO: 1 and the antisense strand is SEQ ID NO: 29, the sense strand is SEQ ID NO: 1 and the antisense strand is SEQ ID NO: 30, the sense strand is SEQ ID NO: 1 and the antisense strand is SEQ ID NO: 31, the sense strand is SEQ ID NO: 1 and the antisense strand is SEQ ID NO: 32, the sense strand is SEQ ID NO: 37 and the antisense strand is SEQ ID NO: 10, the sense strand is SEQ ID NO: 38 and the antisense strand is SEQ ID NO: 10, the sense strand is SEQ ID NO: 3 and the antisense strand is SEQ ID NO: 39, the sense strand is SEQ ID NO: 3 and the antisense strand is SEQ ID NO: 10, and the sense strand is SEQ ID NO: 3 and the antisense strand is SEQ ID NO: 40, and each of the nucleotides and r in the double-stranded siRNA analogue is independently modified or unmodified.

In some embodiments, the double-stranded siRNA analogue is selected from the following:

| Serial number | SEQ ID NO | Sequence of sense strand (5'-3') | SEQ ID NO | Sequence of antisense strand (5'-3') |
|---|---|---|---|---|
| 1 | 3 | GrGUGCACUUCGCUUCACA | 4 | UGUGArGCGAAGUGCACACUU |
| 2 | 5 | GUrUGCACUUCGCUUCACA | 4 | UGUGArGCGAAGUGCACACUU |
| 3 | 3 | GrGUGCACUUCGCUUCACA | 6 | UrUGAAGCGAAGUGCACACUU |
| 4 | 5 | GUrUGCACUUCGCUUCACA | 6 | UrUGAAGCGAAGUGCACACUU |
| 5 | 3 | GrGUGCACUUCGCUUCACA | 7 | UGrGAAGCGAAGUGCACACUU |
| 6 | 5 | GUrUGCACUUCGCUUCACA | 7 | UGrGAAGCGAAGUGCACACUU |
| 7 | 3 | GrGUGCACUUCGCUUCACA | 8 | UGUrAAGCGAAGUGCACACUU |
| 8 | 5 | GUrUGCACUUCGCUUCACA | 8 | UGUrAAGCGAAGUGCACACUU |
| 9 | 3 | GrGUGCACUUCGCUUCACA | 9 | UGUGrAGCGAAGUGCACACUU |
| 10 | 5 | GUrUGCACUUCGCUUCACA | 9 | UGUGrAGCGAAGUGCACACUU |
| 11 | 3 | GrGUGCACUUCGCUUCACA | 10 | UGUGAArCGAAGUGCACACUU |
| 12 | 5 | GUrUGCACUUCGCUUCACA | 10 | UGUGAArCGAAGUGCACACUU |
| 13 | 3 | GrGUGCACUUCGCUUCACA | 11 | UGUGAAGrGAAGUGCACACUU |
| 14 | 5 | GUrUGCACUUCGCUUCACA | 11 | UGUGAAGrGAAGUGCACACUU |
| 15 | 12 | GUGUGCrCUUCGCUUCACA | 4 | UGUGArGCGAAGUGCACACUU |
| 16 | 13 | GUGUGCACUUCGCUUCrCA | 4 | UGUGArGCGAAGUGCACACUU |
| 17 | 14 | GUGUGCACUUCGCUUCACr | 4 | UGUGArGCGAAGUGCACACUU |
| 18 | 1 | GUGUGCACUUCGCUUCACA | 4 | UGUGArGCGAAGUGCACACUU |
| 19 | 1 | GUGUGCACUUCGCUUCACA | 6 | UrUGAAGCGAAGUGCACACUU |
| 20 | 1 | GUGUGCACUUCGCUUCACA | 7 | UGrGAAGCGAAGUGCACACUU |
| 21 | 1 | GUGUGCACUUCGCUUCACA | 8 | UGUrAAGCGAAGUGCACACUU |
| 22 | 1 | GUGUGCACUUCGCUUCACA | 9 | UGUGrAGCGAAGUGCACACUU |
| 23 | 1 | GUGUGCACUUCGCUUCACA | 10 | UGUGAArCGAAGUGCACACUU |
| 24 | 1 | GUGUGCACUUCGCUUCACA | 11 | UGUGAAGrGAAGUGCACACUU |
| 25 | 1 | GUGUGCACUUCGCUUCACA | 29 | UGUGAAGCGrAGUGCACACUU |
| 26 | 1 | GUGUGCACUUCGCUUCACA | 30 | UGUGAAGCGArGUGCACACUU |
| 27 | 1 | GUGUGCACUUCGCUUCACA | 31 | UGUGAAGCGAAGUGCrCACUU |
| 28 | 1 | GUGUGCACUUCGCUUCACA | 32 | UGUGAAGCGAAGUGCACrCUU |
| 29 | 37 | GrGUGCACUUCGCUrCACA | 10 | UGUGAArCGAAGUGCACACUU |
| 30 | 38 | GrGUGCACUUCrCUUCACA | 10 | UGUGAArCGAAGUGCACACUU |

(continued)

| Serial number | SEQ ID NO | Sequence of sense strand (5'-3') | SEQ ID NO | Sequence of antisense strand (5'-3') |
|---|---|---|---|---|
| **31** | **3** | GrGUGCACUUCGCUUCACA | **39** | UGUrAArCGAAGUGCACACUU |
| **32** | **3** | GrGUGCACUUCGCUUCACA | **10** | UGUGAArCGAAGUGCACACUU |
| **33** | **3** | GrGUGCACUUCGCUUCACA | **40** | UGUGAAGCGAArUGCACACUU |

Each of the nucleotides and r in the double-stranded siRNA analogue is independently modified or unmodified.

In some embodiments, the double-stranded siRNA analogue is selected from the following: the sense strand is SEQ ID NO: 16 and the antisense strand is SEQ ID NO: 17, the sense strand is SEQ ID NO: 18 and the antisense strand is SEQ ID NO: 17, the sense strand is SEQ ID NO: 16 and the antisense strand is SEQ ID NO: 19, the sense strand is SEQ ID NO: 18 and the antisense strand is SEQ ID NO: 19, the sense strand is SEQ ID NO: 16 and the antisense strand is SEQ ID NO: 20, the sense strand is SEQ ID NO: 18 and the antisense strand is SEQ ID NO: 20, the sense strand is SEQ ID NO: 16 and the antisense strand is SEQ ID NO: 21, the sense strand is SEQ ID NO: 18 and the antisense strand is SEQ ID NO: 21, the sense strand is SEQ ID NO: 16 and the antisense strand is SEQ ID NO: 22, the sense strand is SEQ ID NO: 18 and the antisense strand is SEQ ID NO: 22, the sense strand is SEQ ID NO: 16 and the antisense strand is SEQ ID NO: 23, the sense strand is SEQ ID NO: 18 and the antisense strand is SEQ ID NO: 23, the sense strand is SEQ ID NO: 16 and the antisense strand is SEQ ID NO: 24, the sense strand is SEQ ID NO: 18 and the antisense strand is SEQ ID NO: 24, the sense strand is SEQ ID NO: 25 and the antisense strand is SEQ ID NO: 17, the sense strand is SEQ ID NO: 26 and the antisense strand is SEQ ID NO: 17, the sense strand is SEQ ID NO: 27 and the antisense strand is SEQ ID NO: 17, the sense strand is SEQ ID NO: 28 and the antisense strand is SEQ ID NO: 17, the sense strand is SEQ ID NO: 28 and the antisense strand is SEQ ID NO: 19, the sense strand is SEQ ID NO: 28 and the antisense strand is SEQ ID NO: 20, the sense strand is SEQ ID NO: 28 and the antisense strand is SEQ ID NO: 21, the sense strand is SEQ ID NO: 28 and the antisense strand is SEQ ID NO: 22, the sense strand is SEQ ID NO: 28 and the antisense strand is SEQ ID NO: 23, the sense strand is SEQ ID NO: 28 and the antisense strand is SEQ ID NO: 24, the sense strand is SEQ ID NO: 28 and the antisense strand is SEQ ID NO: 33, the sense strand is SEQ ID NO: 28 and the antisense strand is SEQ ID NO: 34, the sense strand is SEQ ID NO: 28 and the antisense strand is SEQ ID NO: 35, the sense strand is SEQ ID NO: 28 and the antisense strand is SEQ ID NO: 36, the sense strand is SEQ ID NO: 42 and the antisense strand is SEQ ID NO: 23, the sense strand is SEQ ID NO: 43 and the antisense strand is SEQ ID NO: 23, the sense strand is SEQ ID NO: 16 and the antisense strand is SEQ ID NO: 44, the sense strand is SEQ ID NO: 16 and the antisense strand is SEQ ID NO: 45, the sense strand is SEQ ID NO: 16 and the antisense strand is SEQ ID NO: 46, the sense strand is SEQ ID NO: 16 and the antisense strand is SEQ ID NO: 47, and the sense strand is SEQ ID NO: 16 and the antisense strand is SEQ ID NO: 48.

In some embodiments, the double-stranded siRNA analogue is selected from the following:

| Serial number | SEQ ID NO | Sequence of sense strand (5'-3') | SEQ ID NO | Sequence of antisense strand (5'-3') |
|---|---|---|---|---|
| 1 | **16** | g•r•guGcACUucgcuucaca | **17** | u•G•ugargCGaaguGcAcac•u•u |
| 2 | **18** | g•u•ruGcACUucgcuucaca | **17** | u•G•ugargCGaaguGcAcac•u•u |
| 3 | **16** | g•r•guGcACUucgcuucaca | **19** | u•r•ugaAgCGaaguGcAcac•u•u |
| 4 | **18** | g•u•ruGcACUucgcuucaca | **19** | u•r•ugaAgCGaaguGcAcac•u•u |
| 5 | **16** | g•r•guGcACUucgcuucaca | **20** | u•G•rgaAgCGaaguGcAcac•u•u |
| 6 | **18** | g•u•ruGcACUucgcuucaca | **20** | u•G•rgaAgCGaaguGcAcac•u•u |
| 7 | **16** | g•r•guGcACUucgcuucaca | **21** | u•G•uraAgCGaaguGcAcac•u•u |
| 8 | **18** | g•u•ruGcACUucgcuucaca | **21** | u•G•uraAgCGaaguGcAcac•u•u |
| 9 | **16** | g•r•guGcACUucgcuucaca | **22** | u•G•ugrAgCGaaguGcAcac•u•u |
| 10 | **18** | g•u•ruGcACUucgcuucaca | **22** | u•G•ugrAgCGaaguGcAcac•u•u |
| 11 | **16** | g•r•guGcACUucgcuucaca | **23** | u•G•ugaArCGaaguGcAcac•u•u |
| 12 | **18** | g•u•ruGcACUucgcuucaca | **23** | u•G•ugaArCGaaguGcAcac•u•u |

(continued)

| Serial number | SEQ ID NO | Sequence of sense strand (5'-3') | SEQ ID NO | Sequence of antisense strand (5'-3') |
|---|---|---|---|---|
| 13 | **16** | g•r•guGcACUucgcuucaca | **24** | u•G•ugaAgrGaaguGcAcac•u•u |
| 14 | **18** | g•u•ruGcACUucgcuucaca | **24** | u•G•ugaAgrGaaguGcAcac•u•u |
| 15 | **25** | g•u•guGcrCUucgcuucaca | **17** | u•G•ugargCGaaguGcAcac•u•u |
| 16 | **26** | g•u•guGcACUucgcuucrca | **17** | u•G•ugargCGaaguGcAcac•u•u |
| 17 | **27** | g•u•guGcACUucgcuucacr | **17** | u•G•ugargCGaaguGcAcac•u•u |
| 18 | **28** | g•u•guGcACUucgcuucaca | **17** | u•G•ugargCGaaguGcAcac•u•u |
| 19 | **28** | g•u•guGcACUucgcuucaca | **19** | u•r•ugaAgCGaaguGcAcac•u•u |
| 20 | **28** | g•u•guGcACUucgcuucaca | **20** | u•G•rgaAgCGaaguGcAcac•u•u |
| 21 | **28** | g•u•guGcACUucgcuucaca | **21** | u•G•uraAgCGaaguGcAcac•u•u |
| 22 | **28** | g•u•guGcACUucgcuucaca | **22** | u•G•ugrAgCGaaguGcAcac•u•u |
| 23 | **28** | g•u•guGcACUucgcuucaca | **23** | u•G•ugaArCGaaguGcAcac•u•u |
| 24 | **28** | g•u•guGcACUucgcuucaca | **24** | u•G•ugaAgrGaaguGcAcac•u•u |
| 25 | **28** | g•u•guGcACUucgcuucaca | **33** | u•G•uga(Agn)gCGraguGcAcac•u•u |
| 26 | **28** | g•u•guGcACUucgcuucaca | **34** | u•G•uga(Agn)gCGarguGcAcac•u•u |
| 27 | **28** | g•u•guGcACUucgcuucaca | **35** | u•G•uga(Agn)gCGaaguGcrcac•u•u |
| 28 | **28** | g•u•guGcACUucgcuucaca | **36** | u•G•uga(Agn)gCGaaguGcAcrc•u•u |
| 29 | **42** | g•r•guGcACUucgcurcaca | **23** | u•G•ugaArCGaaguGcAcac•u•u |
| 30 | **43** | g•r•guGcACUucrcuucaca | **23** | u•G•ugaArCGaaguGcAcac•u•u |
| 31 | **16** | g•r•guGcACUucgcuucaca | **44** | u•G•uraArCGaaguGcAcac•u•u |
| 32 | **16** | g•r•guGcACUucgcuucaca | **45** | u•G•uga(Agn)rCGaaguGcAcac•u•u |
| 33 | **16** | g•r•guGcACUucgcuucaca | **46** | u•G•uga(Agn)gCGaaruGcAcac•u•u |
| 35 | **16** | g•r•guGcACUucgcuucaca | **47** | VPu•G•ugaArCGaaguGcAcac•u•u |
| 36 | **16** | g•r•guGcACUucgcuucaca | **48** | VPu•G•uga(Agn)rCGaaguGcAcac•u•u |

In the second aspect, the present disclosure provides a conjugate of a double-stranded siRNA analogue, which comprises the double-stranded siRNA analogue according to the first aspect of the present disclosure and a pharmaceutically acceptable conjugate group conjugated to the double-stranded siRNA analogue.

In some embodiments, the pharmaceutically acceptable conjugate group in the conjugate of the double-stranded siRNA analogue contains 1 to 5 GalNAc (N-acetylgalactosamine) groups. Preferably, the pharmaceutically acceptable conjugate group contains 1, 2, 3, 4 or 5 GalNAc groups. More preferably, the pharmaceutically acceptable conjugate group contains 3 or 4 GalNAc groups.

In some embodiments, the pharmaceutically acceptable conjugate group in the conjugate of the double-stranded siRNA analogue comprises compound group D

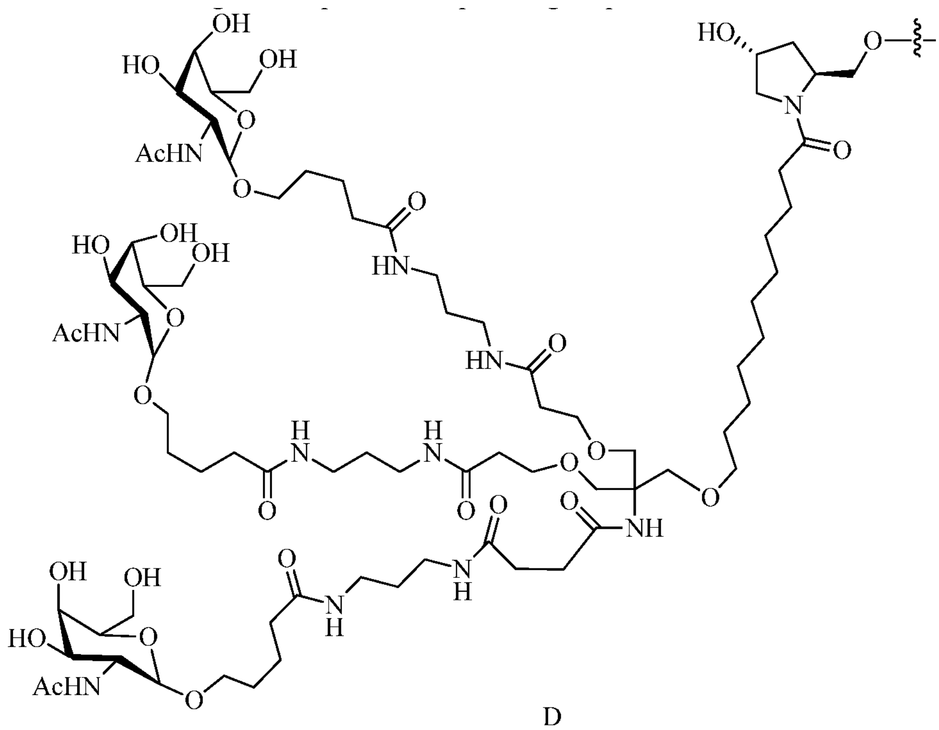

D

In some embodiments, the pharmaceutically acceptable conjugate group in the conjugate of the double-stranded siRNA analogue is linked to the 3' end of the sense strand of the double-stranded siRNA analogue.

In some embodiments, the phosphorothioate moiety of the double-stranded siRNA analogue or the conjugate of the double-stranded siRNA analogue includes (R)- and (S)- enantiomers, diastereoisomers, and/or racemic mixtures thereof.

In some embodiments, the conjugate of the double-stranded siRNA analogue is selected from the following:

| **Serial number** | **Sequence of** sense **strand (5'-3')-conjugate group** | **SEQ ID NO:** | **Sequence of antisense strand (5'-3')** | **SEQ ID NO:** |
|---|---|---|---|---|
| 1 | g•r•guGcACUucgcuucacaD | 16 | u•G•ugargCGaaguGcAcac•u•u | 17 |
| 2 | g•u•ruGcACUucgcuucacaD | 18 | u•G•ugargCGaaguGcAcac•u•u | 17 |
| 3 | g•r•guGcACUucgcuucacaD | 16 | u•r•ugaAgCGaaguGcAcac•u•u | 19 |
| 4 | g•u•ruGcACUucgcuucacaD | 18 | u•r•ugaAgCGaaguGcAcac•u•u | 19 |
| 5 | g•r•guGcACUucgcuucacaD | 16 | u•G•rgaAgCGaaguGcAcac•u•u | 20 |
| 6 | g•u•ruGcACUucgcuucacaD | 18 | u•G•rgaAgCGaaguGcAcac•u•u | 20 |
| 7 | g•r•guGcACUucgcuucacaD | 16 | u•G•uraAgCGaaguGcAcac•u•u | 21 |
| 8 | g•u•ruGcACUucgcuucacaD | 18 | u•G•uraAgCGaaguGcAcac•u•u | 21 |
| 9 | g•r•guGcACUucgcuucacaD | 16 | u•G•ugrAgCGaaguGcAcac•u•u | 22 |
| 10 | g•u•ruGcACUucgcuucacaD | 18 | u•G•ugrAgCGaaguGcAcac•u•u | 22 |
| 11 | g•r•guGcACUucgcuucacaD | 16 | u•G•ugaArCGaaguGcAcac•u•u | 23 |
| 12 | g•u•ruGcACUucgcuucacaD | 18 | u•G•ugaArCGaaguGcAcac•u•u | 23 |
| 13 | g•r•guGcACUucgcuucacaD | 16 | u•G•ugaAgrGaaguGcAcac•u•u | 24 |
| 14 | g•u•ruGcACUucgcuucacaD | 18 | u•G•ugaAgrGaaguGcAcac•u•u | 24 |
| 15 | g•u•guGcrCUucgcuucacaD | 25 | u•G•ugargCGaaguGcAcac•u•u | 17 |
| 16 | g•u•guGcACUucgcuucrcaD | 26 | u•G•ugargCGaaguGcAcac•u•u | 17 |

(continued)

| Serial number | Sequence of sense strand (5'-3')-conjugate group | SEQ ID NO: | Sequence of antisense strand (5'-3') | SEQ ID NO: |
|---|---|---|---|---|
| 17 | g•u•guGcACUucgcuucacrD | 27 | u•G•ugargCGaaguGcAcac•u•u | 17 |
| 18 | g•u•guGcACUucgcuucacaD | 28 | u•G•ugargCGaaguGcAcac•u•u | 17 |
| 19 | g•u•guGcACUucgcuucacaD | 28 | u•r•ugaAgCGaaguGcAcac•u•u | 19 |
| 20 | g•u•guGcACUucgcuucacaD | 28 | u•G•rgaAgCGaaguGcAcac•u•u | 20 |
| 21 | g•u•guGcACUucgcuucacaD | 28 | u•G•uraAgCGaaguGcAcac•u•u | 21 |
| 22 | g•u•guGcACUucgcuucacaD | 28 | u•G•ugrAgCGaaguGcAcac•u•u | 22 |
| 23 | g•u•guGcACUucgcuucacaD | 28 | u•G•ugaArCGaaguGcAcac•u•u | 23 |
| 24 | g•u•guGcACUucgcuucacaD | 28 | u•G•ugaAgrGaaguGcAcac•u•u | 24 |
| 25 | g•u•guGcACUucgcuucacaD | 28 | u•G•uga(Agn) gCGraguGcAcac•u•u | 33 |
| 26 | g•u•guGcACUucgcuucacaD | 28 | u•G•uga(Agn) gCGarguGcAcac•u•u | 34 |
| 27 | g•u•guGcACUucgcuucacaD | 28 | u•G•uga(Agn)gCGaaguGcrcac•u•u | 35 |
| 28 | g•u•guGcACUucgcuucacaD | 28 | u•G•uga(Agn) gCGaaguGcAcrc•u•u | 36 |
| 29 | g•r•guGcACUucgcurcacaD | 42 | u•G•ugaArCGaaguGcAcac•u•u | 23 |
| 30 | g•r•guGcACUucrcuucacaD | 43 | u•G•ugaArCGaaguGcAcac•u•u | 23 |
| 31 | g•r•guGcACUucgcuucacaD | 16 | u•G•uraArCGaaguGcAcac•u•u | 44 |
| 32 | g•r•guGcACUucgcuucacaD | 16 | u•G•uga(Agn) rCGaaguGcAcac•u•u | 45 |
| 33 | g•r•guGcACUucgcuucacaD | 16 | u•G•uga(Agn) gCGaaruGcAcac•u•u | 46 |
| 34 | g•r•guGcACUucgcuucacaD | 16 | VPu•G•ugaArCGaaguGcAcac•u•u | 47 |
| 35 | g•r•guGcACUucgcuucacaD | 16 | VPu•G•uga(Agn) rCGaaguGcAcac•u•u | 48 |

The D is as described above.

In the third aspect, the present disclosure provides a salt of the double-stranded siRNA analogue according to the first aspect of the present disclosure or the conjugate of the double-stranded siRNA analogue according to the second aspect of the present disclosure.

In some embodiments, the salt as described above is selected from a base addition salt, an acid addition salt, and combinations thereof.

In some embodiments, the base addition salt is selected from sodium, potassium, calcium, ammonium, organic amine, magnesium salts and combinations thereof, and the acid addition salt is selected from salts derived from inorganic acids, salts derived from inorganic acids and combinations thereof.

In some embodiments, the inorganic acid is selected from hydrochloric acid, hydrobromic acid, nitric acid, carbonic acid, bicarbonate radical, phosphoric acid, monohydrogen phosphate, dihydrogen phosphate, sulfuric acid, hydrogen sulfate, hydroiodic acid, phosphorous acid and combinations thereof, and the organic acid is selected from acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, methanesulfonic acid and combinations thereof.

In the fourth aspect, the present disclosure provides a pharmaceutical composition, which comprises the double-stranded siRNA analogue according to the first aspect of the present disclosure, the conjugate of the double-stranded siRNA analogue according to the second aspect of the present disclosure or the salt according to the third aspect of the present disclosure, and a pharmaceutically acceptable carrier or excipient.

In the fifth aspect, the present disclosure provides use of the double-stranded siRNA analogue according to the first aspect of the present disclosure, the conjugate of the double-stranded siRNA analogue according to the second aspect of the present disclosure, the salt according to the third aspect of the present disclosure or the pharmaceutical composition according to the fourth aspect of the present disclosure for preparing a medicament for the treatment of hepatitis B.

In some embodiments, the present disclosure provides the double-stranded siRNA analogue according to the first aspect of the present disclosure, the conjugate of the double-stranded siRNA analogue according to the second aspect of the present disclosure, the salt according to the third aspect of the present disclosure or the pharmaceutical composition according to the fourth aspect of the present disclosure, which is used in treating hepatitis B in a subject.

In the sixth aspect, the present disclosure provides a method for treating viral hepatitis B in a subject, which comprises the step of administering to the subject the double-stranded siRNA analogue according to the first aspect of the present disclosure, the conjugate of the double-stranded siRNA analogue according to the second aspect of the present disclosure, the salt according to the third aspect of the present disclosure or the pharmaceutical composition according to the fourth aspect of the present disclosure.

In the seventh aspect, the present disclosure provides the double-stranded siRNA analogue according to the first aspect of the present disclosure, the conjugate of the double-stranded siRNA analogue according to the second aspect of the present disclosure, the salt according to the third aspect of the present disclosure or the pharmaceutical composition according to the fourth aspect of the present disclosure for use in treating hepatitis B in a subject.

In some embodiments of the present disclosure, the hepatitis B may be at any stage of the disease, such as acute hepatitis B, chronic hepatitis B, or cirrhosis or hepatic carcinoma caused by hepatitis B virus infection. In some embodiments, the hepatitis B is chronic hepatitis B.

**Definitions and description**

Unless otherwise stated, the following terms and phrases used herein are intended to have the following meanings. A certain term or phase, unless otherwise specifically defined, should not be considered uncertain or unclear, but construed according to the meaning understood by those of ordinary skill in the art. When referring to a trade name herein, it is intended to refer to its corresponding commercial product or its active ingredient.

In the present disclosure, unless otherwise stated, the terms "comprise", "comprises" and "comprising" or equivalents thereof are open-ended statements and mean that elements, components or steps that are not specified may be included in addition to those listed.

In the present disclosure, HBV gene refers to the gene having a DNA sequence as shown in Genbank Accession No. NC_003977.1. The gene shown as Genbank Accession No. NC_003977.1 is the complete genome of HBV

In some embodiments, a double-stranded siRNA analogue can target the X opening reading frame (X ORF) of HBV

In the present disclosure, a double-stranded siRNA analogue refers to a complex of ribonucleic acid molecules. It has a double-stranded structure, comprises two antiparallel and substantially complementary nucleic acid strands, and has "sense" and "antisense" orientations relative to a target RNA. In the present disclosure, "complementary" has the meaning well known to those skilled in the art. That is, in a double-stranded nucleic acid molecule, bases of one strand pair with bases on the other strand in a complementary manner. A purine base adenine (A) is always paired with a pyrimidine base uracil (U); a purine base guanine (C) is always paired with a pyrimidine base cytosine (G). Each base pair comprises a purine and a pyrimidine. When adenines on one strand are always paired with uracils on the other strand, and guanines are always paired with cytosines, the two strands are considered complementary to each other, and the sequence of the strand can be deduced from the sequence of its complementary strand.

In the present disclosure, unless otherwise specified, C, G, U and A in upper case letters represent the base composition of a nucleotide. c, g, u and a in lower case letters represent the nucleotides represented by the corresponding upper case letters with methoxy modification; the underline _ represents the nucleotides represented by the upper case letters with fluoro modification; the middle dot "•" represents that there is phosphorothioate linkage between two nucleotide residues adjacent to the left and right sides of the middle dot "•"; VP represents that the one nucleotide to the right of the letters VP is an (*E*)-vinyl phosphate modified nucleotide. For example, "a•g" indicates that the a and g residues are linked by a phosphorothioate group.

"Modifications" of nucleotides described in the present disclosure include, but are not limited to, methoxy modification, fluoro modification, (*E*)-vinyl phosphate modification, phosphorothioate linkage, replacement of a nucleotide with (*S*)-glycerol nucleic acids, or the like. The sequences described in the present disclosure may include the sequences listed as "further modified sequences" in Table 1 below.

The fluoro-modified nucleotide described in the present disclosure refers to a nucleotide in which the 2'-hydroxyl of the ribose group is substituted with fluoro, and the methoxy-modified nucleotide refers to a nucleotide in which the 2'-hydroxyl of the ribose group is substituted with methoxy.

The (*E*)-vinyl phosphate modified nucleotides described in the present disclosure represent the following structural unit:

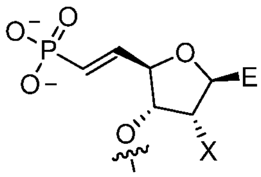,

wherein E is selected from

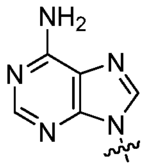, 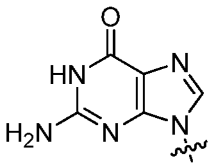, 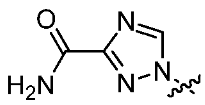, 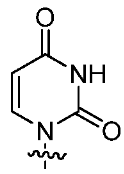

and

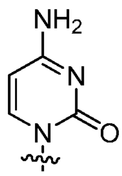;

X is selected from $OCH_3$ and F.

The (*S*)-glycerol nucleic acid (Agn) described in the present disclosure represents the following structural unit:

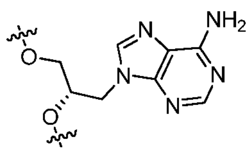

**(Agn)** ;

(Agn) and other nucleotide residues are linked to each other by phosphate or phosphorothioate. For example, "a•(Agn)" represents that a and (Agn) residues are linked by phosphorothioate, and "a(Agn)" represents that a and (Agn) residues are linked by phosphate.

In some embodiments, the double-stranded siRNA analogue comprises a sense strand or an r'-embedded sense strand and an r'-embedded antisense strand. The sense strand, the r'-embedded sense strand and the r'-embedded antisense strand each contain a nucleotide group as a basic structural unit. It is well known to those skilled in the art that nucleotide group comprises a phosphate group, a ribose group and a base, which will not be described in detail herein.

The r'-embedded sequence described in the present disclosure refers to a sequence in which at least one nucleotide residue is linked to r, including sequences obtained by replacing one nucleotide residue in a sequence (e.g., SEQ ID NO: 2) with r. The r'-embedded sequences described in the present disclosure include, but are not limited to: r'-embedded double-stranded siRNA, r'-embedded sense strand and r'-embedded antisense strand. For example, 5'-aGUrrA•C-3', 5'-rGgAAC-3' and 5'-AG•UrAAcCuCr-3' are all r' embedment.

The r'-embedded double-stranded siRNA described in the present disclosure refers to a double-stranded siRNA in which at least one nucleotide residue is linked to r, including double-stranded siRNAs obtained by replacing one nucleotide residue in the sequence of the double-stranded siRNA with r. The r'-embedded sense strand described in the present disclosure refers to a sense strand in which at least one nucleotide residue is linked to r, including the replacement of one or more nucleotides in the sense strand with r. The r'-embedded antisense strand described in the present disclosure refers to an antisense strand in which at least one nucleotide residue is linked to r, including the replacement of one or more nucleotides in the antisense strand with r.

The r' described in the present disclosure is

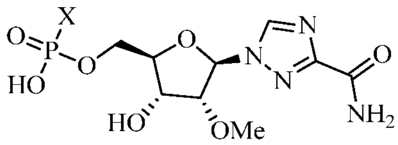

(wherein, X is selected from SH and OH). It is an analogue of a natural nucleotide base and is different from any of the disclosed natural nucleotide bases, and its introduction into the nucleic acid sequence brings about unexpected activity.

The r described in the present disclosure represents the following structural unit:

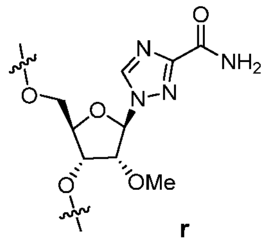

**r** ;

r and other nucleotide residues are linked to each other by phosphate or phosphorothioate. For example, "a•r" represents that a and r residues are linked by phosphorothioate, and "ar" represents that a and r residues are linked by phosphate.

The "a plurality of" described in the present disclosure refers to an integer of 2 or more, including but not limited to 2, 3, 4, 5, 6, 7, 8, 9, 10, 11, 12, 13, 14, 15, 16, 17, 18, 19 or 20, up to the theoretical upper limit of the siRNA analogue.

In the present disclosure, the sense strand or the antisense strand of the double-stranded siRNA analogue may also comprise an "overhang", such as unpaired overhanging nucleotides that are not directly involved in the RNA double helix, wherein the RNA double helix is typically formed by a "sense strand" and "antisense strand" pair as defined herein. Such overhangs may comprise one or more modified or unmodified U, T and A. For example, the SEQ ID NO: 2 may comprise modified or unmodified UU overhangs at the 5' and/or 3' end.

In the present disclosure, the conjugate of the double-stranded siRNA analogue is a compound formed by linking the double-stranded siRNA analogue and a pharmaceutically acceptable conjugate group, and the double-stranded siRNA analogue and the pharmaceutically acceptable conjugate group are covalently linked.

In the present disclosure, a pharmaceutically acceptable conjugate group can be linked to the 3' end of the sense strand or the r'-embedded sense strand of the double-stranded siRNA analogue.

Generally, a pharmaceutically acceptable conjugate group comprises a pharmaceutically acceptable targeting molecule and optionally a linker. Exemplary types of conjugate groups, linkers and targeting molecules can be found in the disclosure of WO2015006740A2. Exemplary conjugate groups include, but are not limited to, L96 or compound group D.

In the context of the present disclosure, unless otherwise stated, "conjugated" means that two or more chemical moieties, each having a particular function, are covalently linked to each other; accordingly, "conjugate" refers to a compound formed by covalent linking of the various chemical moieties.

The compounds of the present disclosure may exist in the form of a specific geometric isomer or stereoisomer. All such compounds are contemplated herein, including (*R*)- and (*S*)- enantiomers, diastereoisomers, and racemic mixtures and other mixtures thereof, such as an enantiomer or diastereoisomer enriched mixture, all of which are encompassed within the scope of the present disclosure. Substituents such as alkyl may have an additional asymmetric carbon atom. All these isomers and mixtures thereof are encompassed within the scope of the present disclosure.

Unless otherwise stated, the term "enantiomer" or "optical isomer" refers to stereoisomers that are mirror images of each other.

Unless otherwise stated, the term "diastereoisomer" refers to stereoisomers whose molecules have two or more chiral centers and are not mirror images of each other.

Unless otherwise stated, the absolute configuration of a stereogenic center is represented by a wedged solid bond (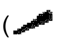) and a wedged dashed bond (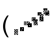), and the relative configuration of a stereogenic center is represented by a straight solid bond (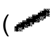) and a straight dashed bond (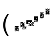). A wavy line (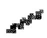) represents a wedged solid bond (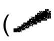) or a wedged dashed bond (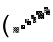), or a wavy line (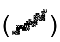) represents a straight solid bond (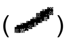) and/or a straight dashed

bond ( 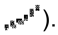 ).

Unless otherwise stated, the term "enriched with one isomer", "isomer enriched", "enriched with one enantiomer", or "enantiomer enriched" means that the content of one of the isomers or enantiomers is less than 100% and more than or equal to 60%, or more than or equal to 70%, or more than or equal to 80%, or more than or equal to 90%, or more than or equal to 95%, or more than or equal to 96%, or more than or equal to 97%, or more than or equal to 98%, or more than or equal to 99%, or more than or equal to 99.5%, or more than or equal to 99.6%, or more than or equal to 99.7%, or more than or equal to 99.8%, or more than or equal to 99.9%.

Unless otherwise stated, the term "isomeric excess" or "enantiomeric excess" refers to the difference between the relative percentages of two isomers or enantiomers. For example, if the content of one of the isomers or enantiomers is 90% and the content of the other isomer or enantiomer is 10%, the isomeric or enantiomeric excess (ee value) is 80%.

Optically active (*R*)- and (*S*)-isomers and *D* and *L* isomers can be prepared by chiral synthesis or chiral reagents or other conventional techniques. An enantiomer of a certain compound of the present disclosure can be prepared by asymmetric synthesis or derivatization using a chiral auxiliary, wherein the resulting diastereoisomeric mixture is separated and the auxiliary group is cleaved so as to provide the desired pure enantiomer. Alternatively, when the molecule contains a basic functional group (such as amino) or an acidic functional group (such as carboxyl), the compound reacts with an appropriate optically active acid or base to form a salt of the diastereoisomer, which is then subjected to diastereoisomeric resolution through conventional methods in the art followed by recovery to give the pure enantiomer. Furthermore, the enantiomer and the diastereoisomer are generally isolated through chromatography using a chiral stationary phase, optionally in combination with chemical derivatization (e.g., carbamate generated from amines). The compound of the present disclosure may contain an unnatural proportion of atomic isotope at one or more of the atoms that constitute the compound. For example, the compound may be labeled with a radioisotope, such as tritium ($^3H$), iodine-125 ($^{125}I$), or C-14 ($^{14}C$). For another example, hydrogen can be substituted with deuterium to form a deuterated drug, and the bond formed by deuterium and carbon is firmer than that formed by common hydrogen and carbon. Compared with an un-deuterated drug, the deuterated drug has the advantages of reduced toxic side effects, increased stability, enhanced efficacy, prolonged biological half-life and the like. All isotopic variations of the compound of the present disclosure, whether radioactive or not, are encompassed within the scope of the present disclosure.

The term "salt" refers to a salt of the compound of the present disclosure, which is prepared from the compound having particular substituents discovered by the present disclosure and a relatively nontoxic acid or base. When the compound of the present disclosure contains a relatively acidic functional group, a base addition salt can be obtained by contacting such a compound with a sufficient amount of a base in a pure solution or a suitable inert solvent. Pharmaceutically acceptable base addition salts include sodium, potassium, calcium, ammonium, organic amine, or magnesium salts, or similar salts. When the compound of the present disclosure contains a relatively basic functional group, an acid addition salt can be obtained by contacting such a compound with a sufficient amount of an acid in a pure solution or a suitable inert solvent. Examples of pharmaceutically acceptable acid addition salts include salts derived from inorganic acids, such as hydrochloric acid, hydrobromic acid, nitric acid, carbonic acid, bicarbonate radical, phosphoric acid, monohydrogen phosphate, dihydrogen phosphate, sulfuric acid, hydrogen sulfate, hydroiodic acid and phosphorous acid or the like; and salts derived from organic acids, such as acetic acid, propionic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, tartaric acid and methanesulfonic acid or the like. Also included are salts of amino acids (e.g., arginine) and salts of organic acids such as glucuronic acid or the like. Certain specific compounds of the present disclosure contain both basic and acidic functional groups that allow the compounds to be converted into either base or acid addition salts.

The salts of the present disclosure can be synthesized from a parent compound having an acidic or basic group using conventional chemical methods. In general, such salts are prepared by the following method: reacting the free acid or base form of the compound with a stoichiometric amount of the appropriate base or acid in water or an organic solvent or a mixture thereof.

The compounds of the present disclosure can be prepared using a variety of synthetic methods which are well known to those skilled in the art, including the specific embodiments listed below, embodiments formed by combinations thereof with other chemical synthetic methods, and equivalents thereof known to those skilled in the art. The preferred embodiments include, but are not limited to, the examples of the present disclosure.

The solvents used in the present disclosure are commercially available.

Unless otherwise specified, the solvent ratios used in column chromatography and preparative thin-layer silica gel chromatography in the present disclosure are volume ratios.

List of acronyms

| | |
|---|---|
| Ac | Acetyl |

(continued)

| | |
|---|---|
| Boc | *Tert*-butyloxycarbonyl |
| DMSO | Dimethyl sulfoxide |
| DMT/DMTr | 4,4'-Dimethoxy triphenylmethyl |
| dsRNA | Double-stranded ribonucleic acid |
| $EC_{50}$ | Half maximal effect concentration |
| EDTA | Ethylenediaminetetraacetic acid disodium salt |
| i-Pr | Isopropyl |
| Me | Methyl |
| Ms | Methanesulfonyl |
| Ph | Phenyl |
| p-HPLC | Preparative high performance liquid chromatography, for the purification of compounds |
| RNA | Ribonucleic acid |
| RNAi | Ribonucleic acid interference technology |
| siRNA | Small interfering ribonucleic acid |
| *t*-Bu | *Tert*-butyl |
| Tris | Tris(hydroxymethyl)aminomethane |

Compounds are named according to conventional nomenclature rules in the art or using ChemDraw® software, and supplier's catalog names are given for commercially available compounds.

## BRIEF DESCRIPTION OF THE DRAWINGS

FIG. 1 shows the effect of WRG01 on HBsAg in plasma of AAV/HBV mice.
FIG. 2 shows the effect of WRG01 on HBeAg in plasma of AAV/HBV mice.
FIG. 3 shows the effect of WRG01 on HBV DNA in plasma of AAV/HBV mice.
FIG. 4 shows the effect of WRG01 on HBV pgRNA in plasma of AAV/HBV mice.
FIG. 5 shows the weight change of mice following WRG01 administration.
FIG. 6 shows the effect of WR007 and WR012 on HBsAg in plasma of AAV/HBV mice.
FIG. 7 shows the effect of WR007 and WR012 on HBeAg in plasma of AAV/HBV mice.
FIG. 8 shows the effect of WR007 and WR012 on HBV DNA in plasma of AAV/HBV mice.
FIG. 9 shows the effect of WRG01 at different doses on HBsAg in plasma of AAV/HBV mice.
FIG. 10 shows the concentration of WRG01 in plasma, liver and kidney of mice.

## DETAILED DESCRIPTION

The present disclosure is described in detail below by way of examples. However, this is by no means disadvantageously limiting the scope of the present disclosure. The compounds of the present disclosure can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present disclosure. It will be apparent to those skilled in the art that various changes and modifications can be made to the specific embodiments of the present disclosure without departing from the spirit and scope of the present disclosure.

### Example 1: Synthesis of Phosphoramidite Monomer

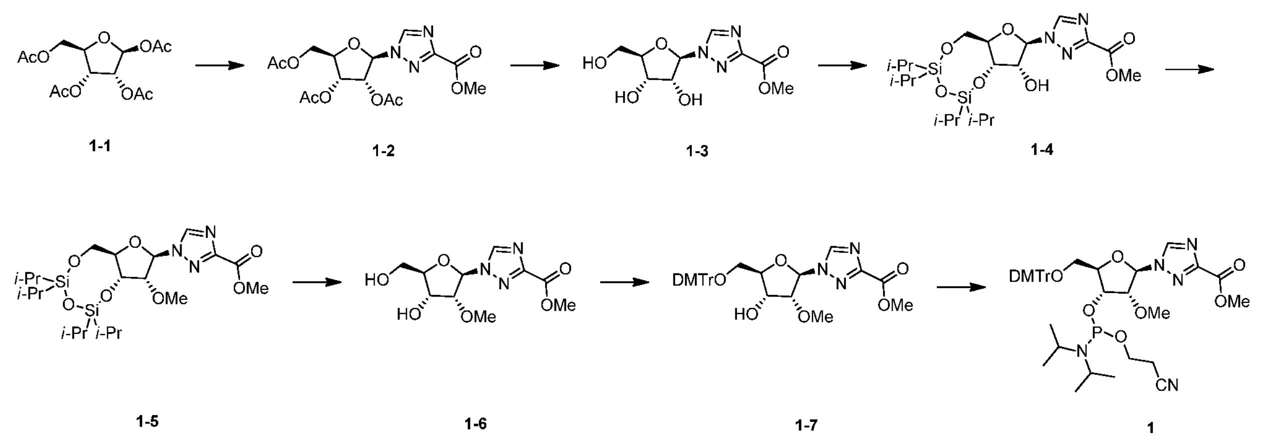

1-1 1-2 1-3 1-4

1-5 1-6 1-7 1

Step A: a solution of (2*S*,3*R*,4*R*,5*R*,6*R*)-3-acetamido-6-(acetoxymethyl)tetrahydro-2*H*-pyran-2,4,5-triyl triacetate (i.e., formula 1-1) (30 g, 94.26 mmol) and methyl 1,2,4-triazole-3-carboxylate (11.98 g, 94.26 mmol) in methyl acetate (220 mL) was concentrated to almost complete dryness in an oil bath at 90 °C under a pressure of 1 bar. A solution of trifluoromethanesulfonic acid (141.46 mg, 0.94 mmol) in methyl acetate (2 mL) was added to the mixture, and the resulting mixture was stirred in an oil bath at 125 °C for 4 h under a pressure of 30 mbar. The reaction solution was cooled to 70 °C, and ethanol (70 mL) was added. The mixture was stirred at 70 °C until a homogeneous solution was formed, and then the stirring was stopped and the solution was cooled to 50 °C. After the precipitate was generated, the reaction solution was left to stand and cooled to 25 °C, and then the reaction solution was left to stand at 0 °C for 16 h. The reaction solution was filtered through a Buchner funnel, and the filter cake was rinsed with 180 mL (60 mL × 3) of ethanol and dried under vacuum to give formula 1-2. $^1H$ NMR (400 MHz, $CDCl_3$): δ 8.40 (s, 1H), 6.04 (d, *J* = 3.42 Hz, 1H), 5.69-5.81 (m, 1H), 5.54 (t, *J* = 5.38 Hz, 1H), 4.42-4.51 (m, 2H), 4.16-4.30 (m, 1H), 3.98 (s, 3H), 2.05-2.18 (m, 9H).

Step B: the compound of formula 1-2 (15 g, 38.93 mmol) and triethylamine (4.14 g, 40.87 mmol) were dissolved in methanol (100 mL). The mixture was stirred at 50 °C for 17 h under nitrogen atmosphere. The reaction solution was concentrated under reduced pressure to give formula 1-3. $^1H$ NMR (400 MHz, $CD_3OD$): δ 8.87 (s, 1H), 5.93 (d, *J* = 3.42 Hz, 1H), 4.48 (dd, *J* = 3.48, 4.83 Hz, 1H), 4.33 (t, *J* = 5.26 Hz, 1H), 4.10-4.16 (m, 1H), 3.95 (s, 3H), 3.84 (dd, *J* = 3.24, 12.29 Hz, 1H), 3.70 (dd, *J* = 4.46, 12.29 Hz, 1H).

Step C: the compound of formula 1-3 (10 g, 38.58 mmol) was dissolved in pyridine (250 mL), and 1,3-dichloro-1,1,3,3-tetraisopropyldisiloxane (12.29 g, 38.97 mmol) was added dropwise at 0 °C. The mixture was gradually warmed to 25 °C and stirred for 16 h. The reaction solution was concentrated under reduced pressure, and the concentrate was suspended in ethyl acetate (250 mL). The mixture was filtered through a Buchner funnel. The filtrate was washed with 750 mL (250 mL × 3) of 3 M hydrochloric acid and 250 mL (250 mL × 1) of saturated brine, dried over anhydrous sodium sulfate, filtered and concentrated under reduced pressure to give a crude product. The crude product was purified by column chromatography ($SiO_2$, petroleum ether/dichloromethane/ethyl acetate = 3/1/1) to give formula 1-4. $^1H$ NMR (400MHz, $CDCl_3$): δ 8.43 (s, 1H), 5.95 (s, 1H), 4.73 (dd, *J* = 4.75, 8.00 Hz, 1H), 4.41 (d, *J* = 4.75 Hz, 1H), 4.09-4.19 (m, 2H), 3.94-4.03 (m, 4H), 2.71-3.34 (m, 1H), 1.01-1.15 (m, 28H).

Step D: iodomethane (11.64 g, 82.02 mmol) was added to a mixed solution of the compound of formula 1-4 (8.23 g, 16.40 mmol), potassium carbonate (11.34 g, 82.02 mmol) and silver(I) oxide (19.01 g, 82.02 mmol) in *N,N*-dimethylformamide (50 mL), and the mixture was stirred at 25 °C for 3 h. The reaction solution was diluted with ethyl acetate (300 mL) and filtered through a Buchner funnel. The filtrate was washed with 250 mL (250 mL × 1) of aqueous sodium thiosulfate solution, 250 mL (250 mL × 1) of water and 250 mL (250 mL × 1) of saturated brine, dried over anhydrous sodium sulfate, filtered and concentrated under reduced pressure to give a crude product. The crude product was purified by column chromatography ($SiO_2$, petroleum ether/ethyl acetate = 5/1) to give formula 1-5. $^1H$ NMR (400 MHz, $CDCl_3$): δ 8.58 (s, 1H), 5.91 (s, 1H), 4.46 (dd, *J* = 4.22, 9.35 Hz, 1H), 4.17-4.28 (m, 2H), 3.96-4.06 (m, 5H), 3.68 (s, 3H), 0.99-1.13 (m, 28H).

Step E: triethylamine trihydrofluoride (2.25 g, 13.95 mmol) was added dropwise to a solution of the compound of formula 1-5 (3.27 g, 6.34 mmol) in tetrahydrofuran (50 mL) at 0 °C, and the mixture was gradually warmed to 25 °C and stirred for 16 h. The reaction solution was concentrated under reduced pressure to give a crude product. The crude product was purified by column chromatography ($SiO_2$, dichloromethane/methanol = 20/1) to give formula 1-6. $^1H$ NMR (400 MHz, $CD_3OD$): δ 8.88 (s, 1H), 6.04 (d, *J* = 3.26 Hz, 1H), 4.44 (t, *J* = 5.33 Hz, 1H), 4.20 (dd, *J* = 3.33, 4.83 Hz, 1H), 4.07-4.14 (m, 1H), 3.96 (s, 3H), 3.84 (dd, *J* = 3.20, 12.36 Hz, 1H), 3.69 (dd, *J* = 4.39, 12.30 Hz, 1H), 3.52 (s, 3H).

Step F: 4,4-dimethoxytrityl chloride (2.42 g, 7.14 mmol) was added to a solution of the compound of formula 1-6 (1.30 g, 4.76 mmol) in pyridine (20 mL) at 0 °C, and the mixture was stirred at 25 °C for 16 h. The reaction solution was diluted with ethyl acetate (70 mL), quenched with saturated aqueous sodium bicarbonate solution (20 mL) at 25 °C

and then diluted with water (40 mL). After liquid separation, the organic phases were combined, washed with 60 mL (60 mL × 1) of water and 60 mL (60 mL × 1) of saturated brine, dried over anhydrous sodium sulfate, filtered and concentrated under reduced pressure to give a crude product. The crude product was purified by p-HPLC (separation column: Phenomenex luna C18 (specification: 250 mm × 50 mm, particle size: 10 μm); mobile phase: [water (10 mM ammonium bicarbonate)-acetonitrile]; elution gradient: 35%-65%, 20 min) to give formula 1-7. $^1H$ NMR (400 MHz, $CDCl_3$): δ 8.44 (s, 1H), 7.38-7.45 (m, 2H), 7.28-7.34 (m, 5H), 7.18-7.27 (m, 2H), 6.70-6.92 (m, 4H), 5.97 (d, *J* = 2.88 Hz, 1H), 4.37-4.43 (m, 1H), 4.33 (dd, *J* = 2.88, 5.00 Hz, 1H), 4.19-4.25 (m, 1H), 3.98 (s, 3H), 3.80 (s, 6H), 3.58 (s, 3H), 3.43-3.49 (m, 1H), 3.33-3.40 (m, 1H), 2.55 (d, *J* = 6.88 Hz, 1H). LCMS (ESI) m/z: 574.2 $[M-H]^-$. Step G: 2-cyanoethyl-*N,N*-diisopropylchlorophosphoramidite (678.45 mg, 2.87 mmol) and *N*,*N*-diisopropylethylamine were added to a solution of the compound of formula 1-7 (1.10 g, 1.91 mmol) in dichloromethane (8 mL) at 0 °C, and the mixture was stirred at 20 °C for 0.5 h. The reaction solution was concentrated under reduced pressure to give a crude product. The crude product was purified by column chromatography ($SiO_2$, petroleum ether/ethyl acetate = 50/1 to 1/2) to give the compound of formula 1. LCMS (ESI) m/z: 776.3 $[M+H]^+$.

**Example 2: Synthesis of D01**

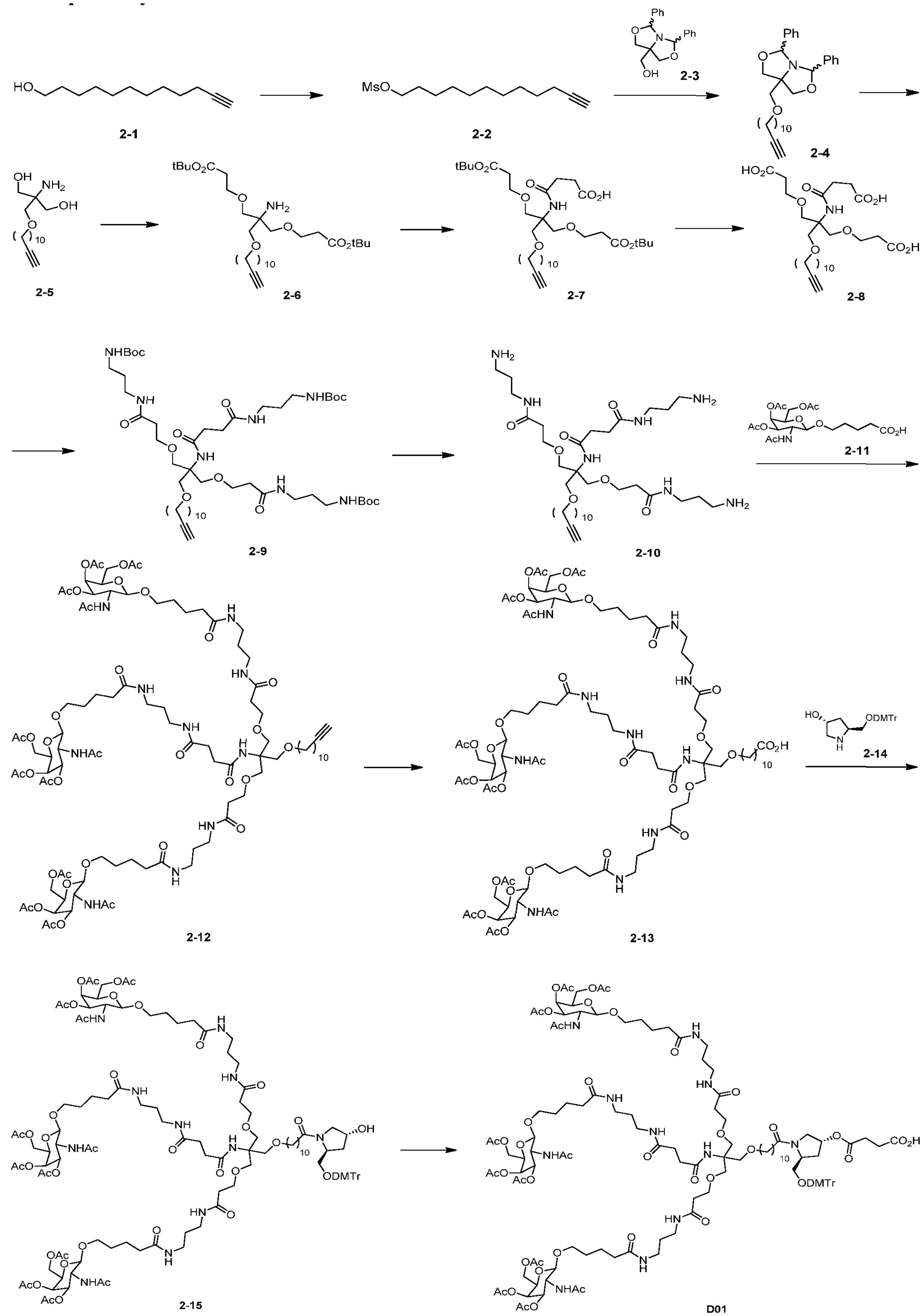


2-1 2-2 2-4

2-5 2-6 2-7 2-8

2-9 2-10

2-12 2-13

2-15 D01

Step A: 11-dodecyn-1-ol (25 g, 137.14 mmol) and triethylamine (16.65 g, 164.56 mmol) were dissolved in dichloromethane (250 mL), and methanesulfonyl chloride (18.85 g, 164.56 mmol) was added at 0 °C. The mixture was stirred at 0 °C for 2 h. The reaction solution was diluted with water (400 mL) and extracted with 800 mL (400 mL × 2) of dichloromethane. The organic phases were combined, washed with 400 mL (200 mL × 2) of water and saturated brine, dried over anhydrous sodium sulfate, filtered and concentrated under reduced pressure to give formula 2-2.

Step B: the compound of formula 2-3 (20 g, 67.26 mmol) was dissolved in *N,N*-dimethylformamide (200 mL), and sodium hydride (60% pure, 4.04 g, 100.89 mmol) was added at 0 °C, followed by the addition of the compound of formula 2-2 (19.27 g, 73.99 mmol). The mixture was stirred at 25 °C for 16 h. The reaction solution was quenched with water (1 L) and extracted with 1.6 L (800 mL × 2) of dichloromethane. The organic phases were combined, washed with 800 mL (800 mL × 1) of saturated brine, dried over anhydrous sodium sulfate, filtered and concentrated under reduced pressure to give formula 2-4. $^1H$ NMR (400 MHz, DMSO-$d_6$): δ 7.63-6.89 (m, 10H), 5.64-5.52 (m, 2H), 4.27-4.01 (m, 2H), 3.98-3.77 (m, 2H), 3.72-3.18 (m, 4H), 2.23-2.14 (m, 2H), 1.98-1.92 (m, 1H), 1.54-1.23 (m, 16H).

Step C: the compound of formula 2-4 (48 g, 103.98 mmol) was dissolved in methanol (870 mL), and a solution of hydrogen chloride in methanol (4 mol/L, 400 mL, 1.6 mol) was added. The mixture was stirred at 30 °C for 2 h. A solution of hydrogen chloride in methanol (4 mol/L, 350 mL, 1.4 mol) was added to the reaction solution. The mixture was stirred at 30 °C for 16 h. The reaction solution was concentrated under reduced pressure, and 200 mL (100 mL × 2) of chloroform was added. The mixture was concentrated under reduced pressure until a white solid appeared. Toluene (130 mL) and petroleum ether (130 mL) were added, and the mixture was stirred at 15 °C for 16 h. The reaction solution was filtered through a Buchner funnel, and the filter cake was collected and dried under vacuum to give a white solid. The white solid was dissolved in dichloromethane (50 mL), and an aqueous solution (50 mL) of sodium hydroxide (6.59 g, 164.66 mmol) was added, and the mixture was stirred at 20 °C for 1 h. The reaction solution was diluted with water (500 mL) and extracted with 1 L (500 mL × 2) of dichloromethane. The organic phases were combined, dried over anhydrous sodium sulfate, filtered and concentrated under reduced pressure to give formula 2-5. Step D: *tert*-butyl acrylate (22.72 g, 177.28 mmol) was added to a mixed solution of the compound of formula 2-5 (23 g, 80.58 mmol) and sodium hydroxide (322.31 mg, 8.06 mmol) in dimethyl sulfoxide (70 mL) and water (6 mL), and the mixture was stirred at 25 °C for 16 h under nitrogen atmosphere. The reaction solution was diluted with water (500 mL) and extracted with 1 L (500 mL × 2) of ethyl acetate. The organic phases were combined, dried over anhydrous sodium sulfate, filtered and concentrated under reduced pressure to give a crude product. The crude product was purified by column chromatography ($SiO_2$, petroleum ether/ethyl acetate/ethanol (containing 0.1% ammonia water) = 36/3/1 to 16/3/1) to give formula 2-6. $^1H$ NMR (400 MHz, DMSO-$d_6$): δ 3.60-3.54 (m, 4H), 3.32 (br s, 5H), 3.15 (s, 5H), 2.74-2.66 (m, 1H), 2.40 (t, *J* = 6.0 Hz, 4H), 2.18-2.11 (m, 2H), 1.58-1.38 (m, 22H), 1.34-1.23 (m, 12H).

Step E: triethylamine (9.15 g, 90.45 mmol) and succinic anhydride (6.79 g, 67.83 mmol) were added to a solution of the compound of formula 2-6 (24.5 g, 45.22 mmol) in dichloromethane (250 mL), and the mixture was stirred at 20 °C for 16 h. Dichloromethane (1 L) and hydrochloric acid (1 mol/L, 1 L) were added to the reaction solution, and after liquid separation, the organic phase was dried over anhydrous sodium sulfate, filtered and concentrated under reduced pressure to give formula 2-7. $^1H$ NMR (400 MHz, $CDCl_3$): δ 6.49-6.37 (m, 1H), 3.72 (s, 2H), 3.70-3.57 (m, 8H), 3.37 (t, *J* = 6.7 Hz, 2H), 2.69-2.51 (m, 4H), 2.50-2.36 (m, 4H), 2.22-2.13 (m, 2H), 1.96-1.90 (m, 1H), 1.57-1.47 (m, 4H), 1.46-1.40 (m, 18H), 1.40-1.31 (m, 2H), 1.30-1.21 (m, 10H).

Step F: the compound of formula 2-7 (27.4 g, 42.69 mmol) was dissolved in formic acid (140 mL), and the mixture was stirred at 20 °C for 16 h under nitrogen atmosphere. The reaction solution was concentrated under reduced pressure, and 300 mL (150 mL × 2) of toluene was added. The mixture was concentrated under reduced pressure to give formula 2-8. $^1H$ NMR (400 MHz, $CDCl_3$): δ 9.79-9.22 (m, 3H), 6.44-6.23 (m, 1H), 3.88-3.43 (m, 10H), 3.39-3.20 (m, 2H), 2.77-2.31 (m, 8H), 2.15-2.06 (m, 2H), 1.87 (t, *J* = 2.6 Hz, 1H), 1.48-1.28 (m, 6H), 1.26-1.12 (m, 10H).

Step G: the compound of formula 2-8 (22.6 g, 42.67 mmol), *N,N*-diisopropylethylamine (33.09 g, 256.03 mmol) and *O*-(7-azabenzotriazol-1-yl)-*N,N,N,N*-tetramethyluronium hexafluorophosphate (51.92 g, 136.55 mmol) were dissolved in *N,N*-dimethylformamide (250 mL), and *tert-butyl* N-(3-aminopropyl)carbamate (29.74 g, 170.69 mmol) was added. The mixture was stirred at 20 °C for 16 h. Dichloromethane (1 L) and hydrochloric acid (1 mol/L, 1 L) were added to the reaction solution, and after liquid separation, the organic phase was washed successively with 1 L (1 L × 1) of water, 1 L (1 L × 1) of aqueous sodium bicarbonate solution and 1 L (1 L × 1) of saturated brine, dried over anhydrous sodium sulfate, filtered and concentrated under reduced pressure to give a crude product. The crude product was purified by column chromatography ($SiO_2$, petroleum ether/ethyl acetate/ethanol = 40/3/1 to 10/3/1) to give formula 2-9. $^1H$ NMR (400 MHz, $CDCl_3$): δ 7.22-6.79 (m, 3H), 6.77-6.44 (m, 1H), 5.45-5.00 (m, 3H), 3.86-3.73 (m, 2H), 3.72-3.63 (m, 4H), 3.62-3.45 (m, 4H), 3.41-3.32 (m, 2H), 3.32-3.20 (m, 6H), 3.19-3.03 (m, 6H), 2.56-2.47 (m, 4H), 2.47-2.39 (m, 4H), 2.21-2.12 (m, 2H), 1.95-1.90 (m, 1H), 1.70-1.57 (m, 6H), 1.56-1.47 (m, 4H), 1.46-1.38 (m, 29H), 1.30-1.25 (m, 10H).

Step H: the compound of formula 2-9 (15 g, 15.03 mmol) was dissolved in dichloromethane (114 mL), and trifluoroacetic acid (38 mL) was added. The mixture was stirred at 20 °C for 16 h. The reaction solution was concentrated under reduced pressure, and 600 mL (250 mL × 3) of a mixture of toluene/acetonitrile = 3/1 was added. The mixture was concentrated under reduced pressure to give formula 2-10 (tris(trifluoroacetate)).

Step I: the compound of formula 2-11 (22.15 g, 49.50 mmol), *N,N*-diisopropylethylamine (7.75 g, 60.00 mmol), 1-hydroxy-7-azabenzotriazole (6.12 g, 45.00 mmol) and *O*-(7-azabenzotriazol-1-yl)-*N,N,N,N*-tetramethyluronium hexafluorophosphate (20.53 g, 54.00 mmol) were dissolved in *N,N*-dimethylformamide (90 mL), and a solution of the compound of formula 2-10 (tris(trifluoroacetate), 15.6 g, 15.00 mmol) and *N,N*-diisopropylethylamine (21.32 g, 165.00 mmol) in *N,N*-dimethylformamide (120 mL) was added to the mixture. The mixture was stirred at 20 °C for 16 h. Dichloromethane (1.2 L) and hydrochloric acid (1 mol/L, 1 L) were added to the reaction solution, and after liquid separation, the organic phase was washed successively with 1 L (1 L × 1) of water, 1 L (1L × 1) of aqueous sodium bicarbonate solution and 1 L (1 L × 1) of saturated brine, dried over anhydrous sodium sulfate, filtered and concentrated under reduced pressure to give a crude product. The crude product was purified by column chromatography ($SiO_2$, dichloromethane/methanol = 100/1 to 10/1 to dichloromethane/ethanol = 1/1) to give formula 2-12. $^1$H NMR (400 MHz, DMSO-$d_6$): δ 7.87-7.66 (m, 9H), 7.09 (s, 1H), 5.21 (d, *J* = 3.4 Hz, 3H), 4.96 (dd, *J* = 3.4, 11.3 Hz, 3H), 4.48 (d, *J* = 8.5 Hz, 3H), 4.06-3.98 (m, 9H), 3.91-3.82 (m, 3H), 3.74-3.66 (m, 3H), 3.58-3.46 (m, 12H), 3.31 (br s, 3H), 3.07-2.98 (m, 12H), 2.71 (t, *J* = 2.6 Hz, 1H), 2.33-2.22 (m, 8H), 2.16-2.12 (m, 2H), 2.10 (s, 9H), 2.04 (br t, *J* = 7.1 Hz, 6H), 1.99 (s, 9H), 1.89 (s, 9H), 1.81-1.74 (m, 9H), 1.54-1.39 (m, 22H), 1.32 (br dd, *J* = 4.5, 6.7 Hz, 2H), 1.24 (s, 10H).

Step J: the compound of formula 2-12 (1.00 g, 0.50 mmol) and N-methyl-*N,N,N*-tri-*n*-octylammonium chloride (20.35 mg, 50.35 μmol) was dissolved in a mixture of acetic acid (2.7 mL) and n-pentane (6.3 mL), and a solution of potassium permanganate (0.40 g, 2.52 mmol) in water (9 mL) was added dropwise to the mixture at 0 °C. The mixture was stirred at 0-15 °C for 2 h. The reaction was quenched with sodium bisulfite (1.27 g), and hydrochloric acid (2 mol/L, 5 mL) and water (30 mL) were added. The mixture was extracted with 120 mL (40 mL × 3) of a chloroform/isopropanol (3/1) mixture. The organic phases were combined, dried over anhydrous sodium sulfate, filtered and concentrated under reduced pressure, and then 180 mL (30 mL × 6) of a toluene/acetonitrile (1/1) mixture was added. The resulting mixture was concentrated under reduced pressure to give formula 2-13. $^1$H NMR (400 MHz, $CD_3OD$): δ 5.34 (d, *J* = 2.9 Hz, 3H), 5.06 (dd, *J* = 3.3, 11.2 Hz, 3H), 4.56 (d, *J* = 8.4 Hz, 3H), 4.19-4.06 (m, 9H), 4.04-3.98 (m, 3H), 3.87 (td, *J* = 5.7, 9.9 Hz, 4H), 3.72-3.64 (m, 9H), 3.57-3.50 (m, 3H), 3.39 (br t, *J* = 6.4 Hz, 2H), 3.22 (q, *J* = 6.4 Hz, 12H), 2.51-2.40 (m, 9H), 2.21 (br t, *J* = 7.3 Hz, 6H), 2.14 (s, 9H), 2.03 (s, 9H), 1.94 (d, *J* = 7.9 Hz, 18H), 1.72-1.57 (m, 22H), 1.39 (br s, 12H).

Step K: *N*, *N*-diisopropylethylamine (0.26 g, 1.99 mmol) and *O*-(7-azabenzotriazol-1-yl)-*N,N,N,N*-tetramethyluronium hexafluorophosphate (0.23 g, 0.60 mmol) were added to a solution of the compound of formula 2-13 (1.00 g, 0.50 mmol) in *N*,*N*-dimethylformamide (10 mL). After the mixture was stirred, the compound of formula 2-14 (0.23 g, 0.55 mmol) was added. The mixture was stirred at 15 °C for 16 h. Dichloromethane (50 mL) and water (50 mL) were added to the reaction solution, and after liquid separation, the organic phase was washed successively with 50 mL (50 mL × 1) of saturated aqueous sodium bicarbonate solution, 50 mL (50 mL × 1) of water and 50 mL (50 mL × 1) of saturated brine, dried over anhydrous sodium sulfate, filtered and concentrated under reduced pressure to give a crude product. The crude product was purified by column chromatography ($SiO_2$, dichloromethane/methanol (containing 0.1% triethylamine) = 20/1 to 10/1) to give formula 2-15. $^1$H NMR (400 MHz, DMSO-$d_6$): δ 7.90-7.82 (m, 6H), 7.78 (br d, *J* = 4.8 Hz, 3H), 7.40-7.26 (m, 10H), 6.91 (br dd, *J* = 3.1, 9.0 Hz, 4H), 5.26 (d, *J* = 3.4 Hz, 3H), 5.03-4.99 (m, 3H), 4.53 (d, *J* = 8.4 Hz, 3H), 4.43 (br d, *J* = 3.8 Hz, 1H), 4.23-4.14 (m, 1H), 4.12-4.02 (m, 9H), 3.92 (td, *J* = 9.0, 11.0 Hz, 3H), 3.78 (s, 6H), 3.77-3.71 (m, 3H), 3.66-3.51 (m, 13H), 3.49-3.41 (m, 4H), 3.11-3.01 (m, 16H), 2.38-2.37 (m, 1H), 2.32 (br s, 9H), 2.14 (s, 9H), 2.08 (br t, *J* = 6.9 Hz, 7H), 2.04 (s, 9H), 1.93 (s, 9H), 1.82 (s, 9H), 1.57-1.46 (m, 22H), 1.31-1.26 (m, 12H).

Step L: triethylamine (67.24 mg, 0.64 mmol), 4-*N,N*-dimethylaminopyridine (0.12 g, 1.00 mmol) and succinic anhydride (83.13 mg, 0.83 mmol) were added successively to a solution of the compound of formula 2-15 (0.80 g, 0.33 mmol) in dichloromethane (8 mL). The mixture was stirred at 10 °C for 16 h. Dichloromethane (50 mL), water (30 mL) and saturated brine (30 mL) were added to the reaction solution, and after liquid separation, the organic phase was washed successively with 30 mL (30 mL × 1) of water and 30 mL (30 mL × 1) of saturated brine, dried over anhydrous sodium sulfate, filtered and concentrated under reduced pressure to obtain a crude product. The crude product was purified by p-HPLC (separation column: Waters Xbridge C18 (specification: 150 mm × 50 mm, particle size: 10 μm); mobile phase: [water (10 mM ammonium bicarbonate)-acetonitrile]; elution gradient: 27% -57%, 11 min) to give Example 2 (compound D01). $^1$H NMR (400 MHz, DMSO-$d_6$): δ 7.96-7.69 (m, 9H), 7.33-7.09 (m, 10H), 6.90-6.78 (m, 4H), 5.21 (d, *J* = 3.3 Hz, 3H), 4.97 (dd, *J* = 3.3, 11.2 Hz, 3H), 4.49 (d, *J* = 8.4 Hz, 3H), 4.06-3.97 (m, 9H), 3.91-3.83 (m, 3H), 3.79-3.66 (m, 11H), 3.63-3.45 (m, 18H), 3.02 (br d, *J* = 4.6 Hz, 14H), 2.46-2.37 (m, 4H), 2.35-2.14 (m, 12H), 2.10 (s, 9H), 2.04 (br t, *J* = 7.0 Hz, 6H), 1.99 (s, 9H), 1.88 (s, 9H), 1.77 (s, 9H), 1.57-1.37 (m, 22H), 1.22 (br s, 12H).

**Example 3: Synthesis of Double-Stranded siRNA Analogue or Conjugates Thereof**

Synthesis of D-containing single-stranded oligoribonucleotides: oligoribonucleotides were synthesized according to the phosphoramidite solid-phase synthesis technique. Synthesis was performed on a solid support made by covalently linking controlled porous glass (amino CPG, 500 Å) to D01. All 2'-modified RNA phosphoramidites and ancillary reagents were commercially available reagents. All amides were dissolved in anhydrous acetonitrile and a molecular sieve (3 Å) was added, and the coupling time when using 5-ethylthio-1*H*-tetrazole (ETT) as the activating agent was 5

min. Phosphorothioate bonds were generated using a 50 mM solution of 3-((dimethylamino-methylene)amino)-3*H*-1,2,4-dithiazole-3-thione (DDTT) in anhydrous acetonitrile/pyridine (v/v = 1/1), and the reaction time was 3 min. The sequences were synthesized after the removal of the DMT group at last.

Synthesis of D-free single-stranded oligoribonucleotides: oligoribonucleotides were synthesized according to the phosphoramidite solid-phase synthesis technique. The synthesis was performed on universal controlled porous glass CPG (500 Å). All 2'-modified RNA phosphoramidites and ancillary reagents were commercially available reagents. All amides were dissolved in anhydrous acetonitrile and a molecular sieve (3 Å) was added, and the coupling time when using 5-ethylthio-1*H*-tetrazole (ETT) as the activating agent was 5 min. Phosphorothioate bonds were generated using a 50 mM solution of 3-((dimethylamino-methylene)amino)-3*H*-1,2,4-dithiazole-3-thione (DDTT) in anhydrous acetonitrile/pyridine (v/v = 1/1), and the reaction time was 3 min. The sequences were synthesized after the removal of the DMT group at last.

Cleavage and deprotection of bound oligomers on CPG: after the solid-phase synthesis was terminated, the protecting group was removed by treatment with a solution of 20% diethylamine in acetonitrile for 30 min, without cleaving the oligonucleotide from the CPG. Subsequently, the dried CPG was treated with concentrated ammonia water at 40 °C for 18 h. After centrifugation, the supernatant was transferred to a new tube and the CPG was washed with ammonia water. The combined solution was concentrated to give a solid mixture.

Purification of single-stranded oligoribonucleotides: the oligomers purified by HPLC were exchanged by using NanoQ anions. Buffer A was a 10 mM sodium perchlorate solution, 20 mM Tris, 1 mM EDTA, pH 7.4 and containing 20% acetonitrile, and buffer B was 500 mM sodium perchlorate, 20 mM Tris, 1 mM EDTA, pH 7.4 and containing 20% acetonitrile. The desired product was separated out and desalted using a reverse phase C18 column.

Annealing of single-stranded oligoribonucleotides for siRNA production: the single-stranded oligoribonucleotides to be annealed were formulated to 200 μM using sterile RNase Free $H_2O$ (no RNA hydrolase). The annealing reaction system was set as follows: a total of 100 μL of 10 nmol mixed solution was placed in 95 °C water bath for 10 min (20 min at high temperature was required for 100 nmol or more) → the solution was quickly placed in 60 °C water bath, and naturally cooled → the solution after annealing cannot be placed at high temperature for storage. Complementary strands were formed by combining equimolar solutions of single-stranded oligoribonucleotides.

**Table 1. Double-stranded siRNA analogues targeting hepatitis B virus genes, conjugates comprising the same, and their corresponding core sequences**

| Core sequences | | | | r'-embedded sequences** | | | | Further modified sequences | | | |
|---|---|---|---|---|---|---|---|---|---|---|---|
| SEQ ID NO | Sequence of sense strand (5'-3') | SEQ ID NO | Sequence of antisense strand (5'-3') | SEQ ID NO | Sequence of sense strand (5'-3')*** | SEQ ID NO | Sequence of antisense strand (5'-3') | SEQ ID NO | Sequence of sense strand (5'-3')*** | SEQ ID NO | Sequence of antisense strand (5'-3') |
| 1 | GUGUGCACUUCGCUUCACA | 2 | UGUGAAGCGAAGUGCACAC | 3 | GrGUGCACUUCGCUUCACAD* | 4 | UGUGArGCGAAGUGCACACUU | 16 | g•r•guGcACUucgcuucacaD* | 17 | u•G•ugargCGaaguGcAcac•u•u |
| 1 | GUGUGCACUUCGCUUCACA | 2 | UGUGAAGCGAAGUGCACAC | 5 | GUrUGCACUUCGCUUCACAD | 4 | UGUGArGCGAAGUGCACACUU | 18 | g•u•ruGcACUucgcuucacaD | 17 | u•G•ugargCGaaguGcAcac•u•u |
| 1 | GUGUGCACUUCGCUUCACA | 2 | UGUGAAGCGAAGUGCACAC | 3 | GrGUGCACUUCGCUUCACAD | 6 | UrUGAAGCGAAGUGCACACUU | 16 | g•r•guGcACUucgcuucacaD | 19 | u•r•ugaAgCGaaguGcAcac•u•u |
| 1 | GUGUGCACUUCGCUUCACA | 2 | UGUGAAGCGAAGUGCACAC | 5 | GUrUGCACUUCGCUUCACAD | 6 | UrUGAAGCGAAGUGCACACUU | 18 | g•u•ruGcACUucgcuucacaD | 19 | u•r•ugaAgCGaaguGcAcac•u•u |
| 1 | GUGUGCACUUCGCUUCACA | 2 | UGUGAAGCGAAGUGCACAC | 3 | GrGUGCACUUCGCUUCACAD | 7 | UGrGAAGCGAAGUGCACACUU | 16 | g•r•guGcACUucgcuucacaD | 20 | u•G•rgaAgCGaaguGcAcac•u•u |
| 1 | GUGUGCACUUCGCUUCACA | 2 | UGUGAAGCGAAGUGCACAC | 5 | GUrUGCACUUCGCUUCACAD | 7 | UGrGAAGCGAAGUGCACACUU | 18 | g•u•ruGcACUucgcuucacaD | 20 | u•G•rgaAgCGaaguGcAcac•u•u |
| 1 | GUGUGCACUUCGCUUCACA | 2 | UGUGAAGCGAAGUGCACAC | 3 | GrGUGCACUUCGCUUCACAD | 8 | UGUrAAGCGAAGUGCACACUU | 16 | g•r•guGcACUucgcuucacaD | 21 | u•G•uraAgCGaaguGcAcac•u•u |
| 1 | GUGUGCACUUCGCUUCACA | 2 | UGUGAAGCGAAGUGCACAC | 5 | GUrUGCACUUCGCUUCACAD | 8 | UGUrAAGCGAAGUGCACACUU | 18 | g•u•ruGcACUucgcuucacaD | 21 | u•G•uraAgCGaaguGcAcac•u•u |
| 1 | GUGUGCACUUCGCUUCACA | 2 | UGUGAAGCGAAGUGCACAC | 3 | GrGUGCACUUCGCUUCACAD | 9 | UGUGrAGCGAAGUGCACACUU | 16 | g•r•guGcACUucgcuucacaD | 22 | u•G•ugrAgCGaaguGcAcac•u•u |

(continued)

| Core sequences | | | | r'-embedded sequences** | | | | Further modified sequences | | | |
|---|---|---|---|---|---|---|---|---|---|---|---|
| SEQ ID NO | Sequence of sense strand (5'-3') | SEQ ID NO | Sequence of antisense strand (5'-3') | SEQ ID NO | Sequence of sense strand (5'-3')*** | SEQ ID NO | Sequence of antisense strand (5'-3') | SEQ ID NO | Sequence of sense strand (5'-3')*** | SEQ ID NO | Sequence of antisense strand (5'-3') |
| 1 | GUGUGCACUUCGCUUCACA | 2 | UGUGAAGCGAAGUGCACAC | 5 | GUrUGCACUUCGCUUCACAD | 9 | UGUGrAGCGAAGUGCACACUU | 18 | g•u•ruGcACUucgcuucacaD | 22 | u•G•ugrAgCGaaguGcAcac•u•u |
| 1 | GUGUGCACUUCGCUUCACA | 2 | UGUGAAGCGAAGUGCACAC | 3 | GrGUGCACUUCGCUUCACAD | 10 | UGUGAArCGAAGUGCACACUU | 16 | g•r•guGcACUucgcuucacaD | 23 | u•G•ugaArCGaaguGcAcac•u•u |
| 1 | GUGUGCACUUCGCUUCACA | 2 | UGUGAAGCGAAGUGCACAC | 5 | GUrUGCACUUCGCUUCACAD | 10 | UGUGAArCGAAGUGCACACUU | 18 | g•u•ruGcACUucgcuucacaD | 23 | u•G•ugaArCGaaguGcAcac•u•u |
| 1 | GUGUGCACUUCGCUUCACA | 2 | UGUGAAGCGAAGUGCACAC | 3 | GrGUGCACUUCGCUUCACAD | 11 | UGUGAAGrGAAGUGCACACUU | 16 | g•r•guGcACUucgcuucacaD | 24 | u•G•ugaAgrGaaguGcAcac•u•u |
| 1 | GUGUGCACUUCGCUUCACA | 2 | UGUGAAGCGAAGUGCACAC | 5 | GUrUGCACUUCGCUUCACAD | 11 | UGUGAAGrGAAGUGCACACUU | 18 | g•u•ruGcACUucgcuucacaD | 24 | u•G•ugaAgrGaaguGcAcac•u•u |
| 1 | GUGUGCACUUCGCUUCACA | 2 | UGUGAAGCGAAGUGCACAC | 12 | GUGUGCrCUUCGCUUCACAD | 4 | UGUGArGCGAAGUGCACACUU | 25 | g•u•guGcrCUucgcuucacaD | 17 | u•G•ugargCGaaguGcAcac•u•u |
| 1 | GUGUGCACUUCGCUUCACA | 2 | UGUGAAGCGAAGUGCACAC | 13 | GUGUGCACUUCGCUUCrCAD | 4 | UGUGArGCGAAGUGCACACUU | 26 | g•u•guGcACUucgcuucrcaD | 17 | u•G•ugargCGaaguGcAcac•u•u |
| 1 | GUGUGCACUUCGCUUCACA | 2 | UGUGAAGCGAAGUGCACAC | 14 | GUGUGCACUUCGCUUCACrD | 4 | UGUGArGCGAAGUGCACACUU | 27 | g•u•guGcACUucgcuucacrD | 17 | u•G•ugargCGaaguGcAcac•u•u |
| 1 | GUGUGCACUUCGCUUCACA | 2 | UGUGAAGCGAAGUGCACAC | 1 | GUGUGCACUUCGCUUCACAD | 4 | UGUGArGCGAAGUGCACACUU | 28 | g•u•guGcACUucgcuucacaD | 17 | u•G•ugargCGaaguGcAcac•u•u |

(continued)

| Core sequences | | | | r'-embedded sequences** | | | | Further modified sequences | | | |
|---|---|---|---|---|---|---|---|---|---|---|---|
| SEQ ID NO | Sequence of sense strand (5'-3') | SEQ ID NO | Sequence of antisense strand (5'-3') | SEQ ID NO | Sequence of sense strand (5'-3')*** | SEQ ID NO | Sequence of antisense strand (5'-3') | SEQ ID NO | Sequence of sense strand (5'-3')*** | SEQ ID NO | Sequence of antisense strand (5'-3') |
| 1 | GUGUGCACUUCGCUUCACA | 2 | UGUGAAGCGAAGUGCACAC | 1 | GUGUGCACUUCGCUUCACAD | 6 | UrUGAAGCGAAGUGCACACUU | 28 | g•u•guGcACUucgcuucacaD | 19 | u•r•ugaAgCGaaguGcAcac•u•u |
| 1 | GUGUGCACUUCGCUUCACA | 2 | UGUGAAGCGAAGUGCACAC | 1 | GUGUGCACUUCGCUUCACAD | 7 | UGrGAAGCGAAGUGCACACUU | 28 | g•u•guGcACUucgcuucacaD | 20 | u•G•rgaAgCGaaguGcAcac•u•u |
| 1 | GUGUGCACUUCGCUUCACA | 2 | UGUGAAGCGAAGUGCACAC | 1 | GUGUGCACUUCGCUUCACAD | 8 | UGUrAAGCGAAGUGCACACUU | 28 | g•u•guGcACUucgcuucacaD | 21 | u•G•uraAgCGaaguGcAcac•u•u |
| 1 | GUGUGCACUUCGCUUCACA | 2 | UGUGAAGCGAAGUGCACAC | 1 | GUGUGCACUUCGCUUCACAD | 9 | UGUGrAGCGAAGUGCACACUU | 28 | g•u•guGcACUucgcuucacaD | 22 | u•G•ugrAgCGaaguGcAcac•u•u |
| 1 | GUGUGCACUUCGCUUCACA | 2 | UGUGAAGCGAAGUGCACAC | 1 | GUGUGCACUUCGCUUCACAD | 10 | UGUGAArCGAAGUGCACACUU | 28 | g•u•guGcACUucgcuucacaD | 23 | u•G•ugaArCGaaguGcAcac•u•u |
| 1 | GUGUGCACUUCGCUUCACA | 2 | UGUGAAGCGAAGUGCACAC | 1 | GUGUGCACUUCGCUUCACAD | 11 | UGUGAAGrGAAGUGCACACUU | 28 | g•u•guGcACUucgcuucacaD | 24 | u•G•ugaAgrGaaguGcAcac•u•u |
| 1 | GUGUGCACUUCGCUUCACA | 2 | UGUGAAGCGAAGUGCACAC | 1 | GUGUGCACUUCGCUUCACAD | 29 | UGUGAAGCGrAGUGCACACUU | 28 | g•u•guGcACUucgcuucacaD | 33 | u•G•uga(Agn)gCGraguGcAcac•u•u |

(continued)

| Core sequences | | | | r'-embedded sequences** | | | | Further modified sequences | | | |
|---|---|---|---|---|---|---|---|---|---|---|---|
| SEQ ID NO | Sequence of sense strand (5'-3') | SEQ ID NO | Sequence of antisense strand (5'-3') | SEQ ID NO | Sequence of sense strand (5'-3')*** | SEQ ID NO | Sequence of antisense strand (5'-3') | SEQ ID NO | Sequence of sense strand (5'-3')*** | SEQ ID NO | Sequence of antisense strand (5'-3') |
| 1 | GUGUGCA CUUCGCU UCACA | 2 | UGUGAAG CGAAGUG CACAC | 1 | GUGUGC ACUUCGC UUCACA D | 30 | UGUGAAG CGArGUG CACACUU | 28 | g•u•guGcA CUucgcuuc acaD | 34 | u•G•uga(Agn )gCGarguGc Acac•u•u |
| 1 | GUGUGCA CUUCGCU UCACA | 2 | UGUGAAG CGAAGUG CACAC | 1 | GUGUGC ACUUCGC UUCACA D | 31 | UGUGAAG CGAAGUG CrCACUU | 28 | g•u•guGcA CUucgcuuc acaD | 35 | u•G•uga(Agn )gCGaaguGcr cac•u•u |
| 1 | GUGUGCA CUUCGCU UCACA | 2 | UGUGAAG CGAAGUG CACAC | 1 | GUGUGC ACUUCGC UUCACA D | 32 | UGUGAAG CGAAGUG CACrCUU | 28 | g•u•guGcA CUucgcuuc acaD | +36 | u•G•uga(Agn )gCGaaguGc Acrc•u•u |
| 1 | GUGUGCA CUUCGCU UCACA | 2 | UGUGAAG CGAAGUG CACAC | 37 | GrGUGCA CUUCGCU rCACAD | 10 | UGUGAAr CGAAGUG CACACUU | 42 | g•r•guGcA CUucgcurc acaD | 23 | u•G•ugaArC GaaguGcAca c•u•u |
| 1 | GUGUGCA CUUCGCU UCACA | 2 | UGUGAAG CGAAGUG CACAC | 38 | GrGUGCA CUUCrCU UCACAD | 10 | UGUGAAr CGAAGUG CACACUU | 43 | g•r•guGcA CUucrcuuc acaD | 23 | u•G•ugaArC GaaguGcAca c•u•u |
| 1 | GUGUGCA CUUCGCU UCACA | 2 | UGUGAAG CGAAGUG CACAC | 3 | GrGUGCA CUUCGCU UCACAD | 39 | UGUrAArC GAAGUGC ACACUU | 16 | g•r•guGcA CUucgcuuc acaD | 44 | u•G•uraArCG aaguGcAcac• u•u |
| 1 | GUGUGCA CUUCGCU UCACA | 2 | UGUGAAG CGAAGUG CACAC | 3 | GrGUGCA CUUCGCU UCACAD | 10 | UGUGAAr CGAAGUG CACACUU | 16 | g•r•guGcA CUucgcuuc acaD | 45 | u•G•uga(Agn )rCGaaguGc Acac•u•u |
| 1 | GUGUGCA CUUCGCU UCACA | 2 | UGUGAAG CGAAGUG CACAC | 3 | GrGUGCA CUUCGCU UCACAD | 40 | UGUGAAG CGAArUG CACACUU | 16 | g•r•guGcA CUucgcuuc acaD | 46 | u•G•uga(Agn )gCGaaruGc Acac•u•u |

(continued)

| Core sequences | | | | r'-embedded sequences** | | | | Further modified sequences | | | |
|---|---|---|---|---|---|---|---|---|---|---|---|
| SEQ ID NO | Sequence of sense strand (5'-3') | SEQ ID NO | Sequence of antisense strand (5'-3') | SEQ ID NO | Sequence of sense strand (5'-3')*** | SEQ ID NO | Sequence of antisense strand (5'-3') | SEQ ID NO | Sequence of sense strand (5'-3')*** | SEQ ID NO | Sequence of antisense strand (5'-3') |
| 1 | GUGUGCA CUUCGCU UCACA | 2 | UGUGAAG CGAAGUG CACAC | 3 | GrGUGCA CUUCGCU UCACAD | 10 | UGUGAAr CGAAGUG CACACUU | 16 | g•r•guGcA CUucgcuuc acaD | 47 | VPu•G•ugaA rCGaaguGcA cac•u•u |
| 1 | GUGUGCA CUUCGCU UCACA | 2 | UGUGAAG CGAAGUG CACAC | 3 | GrGUGCA CUUCGCU UCACAD | 10 | UGUGAAr CGAAGUG CACACUU | 16 | g•r•guGcA CUucgcuuc acaD | 48 | VPu•G•uga( Agn)rCGaagu GcAcac•u•u |

(continued)

| Core sequences | | | | r'-embedded sequences** | | | | Further modified sequences | | | |
|---|---|---|---|---|---|---|---|---|---|---|---|
| SEQ ID NO | Sequence of sense strand (5'-3') | SEQ ID NO | Sequence of antisense strand (5'-3') | SEQ ID NO | Sequence of sense strand (5'-3')*** | SEQ ID NO | Sequence of antisense strand (5'-3') | SEQ ID NO | Sequence of sense strand (5'-3')*** | SEQ ID NO | Sequence of antisense strand (5'-3') |
| 1 | GUGUGCACUUCGCUUCACA | 2 | UGUGAAGCGAAGUGCACAC | 3 | GrGUGCACUUCGCUUCACAD | 10 | UGUGAArCGAAGUGCACACUU | 15 | g•u•guGcACUucgcuuracaD | 41 | VPu•G•uga(Agn)gCGarguGcAcac•u•u |

*: D is a residue obtained after chemical reaction of the small molecular fragment D01, is combined with nucleic acid through a covalent bond, and has the following structure:

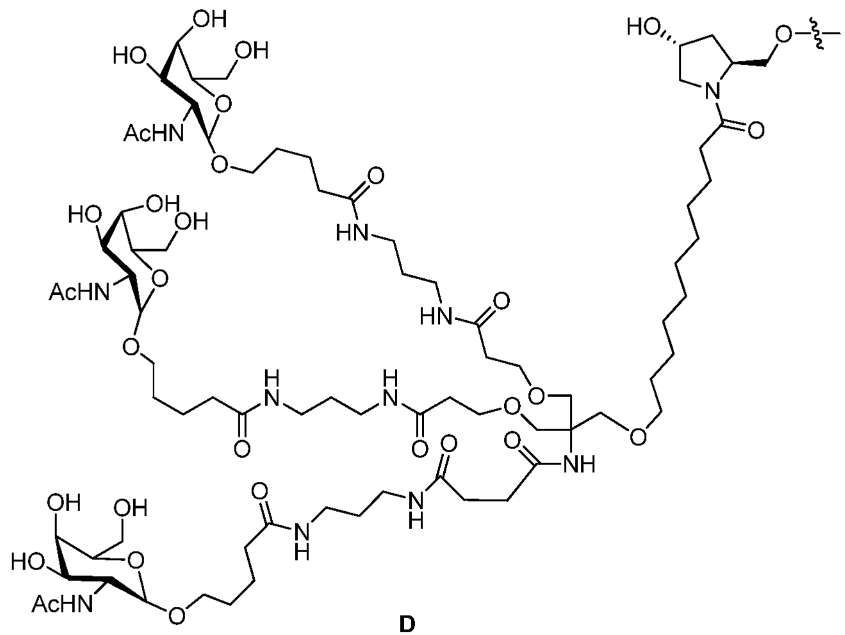

D

**: The sequence of antisense strand in the r'-embedded sequences is obtained by r' embedment on the basis of the sequence of antisense strand with UU at the 3' end in the core sequences. For example, SEQ ID NO: 4 is obtained by r' embedment on the basis of SEQ ID NO: 2 with UU at the 3' end.

***: When a sequence contains D, the D is used to refer to the linking position of the conjugate group D. For example, g•r•guGcACUucgcuucacaD (5'-3') indicates that the sequence set forth in SEQ ID NO. 16, g•r•guGcACUucgcuucaca, is linked to D at the 3' end.

**Example 3: *In Vitro* HBV Assay**

**1. Experimental objective:**

The content of HBV antigens (HBsAg and HBeAg) in HepG2-NTCP cell culture supernatant was detected by enzyme-linked immunosorbent assay (ELISA), and the inhibitory activity of the compound on HBV was evaluated by taking the $EC_{50}$ value of the compound as an index; meanwhile, the cell viability was detected by Cell-titer Glo to evaluate the cytotoxicity of the compound.

**2. Experimental materials:**

2.1. Cell line: HepG2-NTCP cells
HepG2-NTCP cell culture medium (DMEM, Invitrogen-11330032; 10% serum, Invitrogen-10099141; 100 units/mL penicillin and 100 μg/mL streptomycin, Hyclone-SV30010; 1% nonessential amino acids, Invitrogen-11140050; 2 mM L-glutamine, Invitrogen-25030081; 1 mM sodium pyruvate, Gibco-11360-070; 500 μg/mL Geneticin, Invitrogen-10131027)
2.2. Reagents:
Pancreatin (Invitrogen-25300062); DPBS (Corning-21031CVR); DMSO (Sigma-D2650-100 ML); Cell-titer Glo (Promega-G7573); HBsAg quantitative assay kit (Autobio-CL 0310); HBeAg quantitative assay kit (Autobio-CL 0312).
2.3. Consumables and instrument:
96-well cell culture plates (Corning-3599); $CO_2$ incubator (HERA-CELL-240) microplate reader (BioTek Synergy 2)

**3. Experimental procedures and method:**

3.1. On day 0, HepG2-NTCP ($7.5\times10^4$ cells/well) cells were plated onto a 48-well plate and incubated overnight at 37 °C and 5% $CO_2$.
3.2. On day 1, the medium containing 1% DMSO was used for medium change.
3.3. On day 2, HepG2-NTCP (2000 GE/cell) was infected with HBV/D (concentrated from HepG2.2.15 cell culture supernatant).
3.4. On day 3, the infection solution was pipetted off and fresh medium containing 1% DMSO was added.
3.5. On day 6, the siRNA conjugate was transfected according to instructions of Lipofectamine® RNAiMax (Invitrogen). The conjugate was subjected to 5-fold gradient dilution to obtain 7 concentrations, triplicate wells were set, and the final concentration was 0.16 pM. The compound was a combination of sense and antisense strands and was a single chemical entity, with a maximum concentration of 2.5 nM.
3.6. On day 12, supernatant from the culture wells was collected and assayed for HBsAg and HBeAg by ELISA. After the supernatant was collected, Cell-titer Glo was added to measure cell viability.
3.7. Reference was made to the instructions of the product for specific procedures of ELISA assay for HBsAg and HBeAg, and the brief procedures are as follows: 50 μL of sample and 50 μL of standard substance were each added into a reaction plate, then enzyme conjugate was added at 50 μL/well, and the mixture was well mixed by shaking and incubated at 37 °C for 60 min; the plate was washed 5 times by using a washing solution, luminescent substrate was then added at 50 μL/well, and the mixture was well mixed and reacted at room temperature in the dark for 10 min, and finally the chemiluminescence intensity was detected by using a microplate reader.
3.8. Data analysis:

Calculation of percentage cell viability:

% viability = (luminescence value of sample – luminescence value of medium control)/(luminescence value of DMSO control – luminescence value of medium control) × 100.

Calculation of the inhibition percentage for HBsAg and HBeAg:

$$\% \text{ Inh.} = (1 - \text{antigen value in sample/antigen value in DMSO control}) \times 100.$$

Calculation of $CC_{50}$ and $EC_{50}$: $CC_{50}$ and 50% inhibitory concentration for HBV ($EC_{50}$) values of compounds were calculated using GraphPad Prism software.

**4. Experimental results:** see Table 2.

**Table 2. Results of test sequences in reducing HBsAg and HBeAg levels in cells**

| Test sequences | | | | Experimental results | | |
|---|---|---|---|---|---|---|
| SEQ ID NO | Sequence of sense strand (5'-3') | SEQ ID NO | Sequence of antisense strand (5'-3') | HBsAg $EC_{50}$ (pM) | HBeAg $EC_{50}$ (pM) | Cell viability $CC_{50}$ (nM) |
| **16** | g•r•guGcACUucgcuuc acaD | **17** | u•G•ugargCGaaguGcA cac•u•u | 13.75 | 20.84 | >2.5 |
| **16** | g•r•guGcACUucgcuuc acaD | **20** | u•G•rgaAgCGaaguGcA cac•u•u | 17.40 | 34.21 | >2.5 |
| **16** | g•r•guGcACUucgcuuc acaD | **23** | u•G•ugaArCGaaguGcA cac•u•u | 12.44 | 21.07 | >2.5 |
| **28** | g•u•guGcACUucgcuuc acaD | **17** | u•G•ugargCGaaguGcA cac•u•u | 13.09 | 22.68 | >2.5 |
| **28** | g•u•guGcACUucgcuuc acaD | **20** | u•G•rgaAgCGaaguGcA cac•u•u | 14.69 | 28.90 | >2.5 |
| **28** | g•u•guGcACUucgcuuc acaD | **23** | u•G•ugaArCGaaguGcA cac•u•u | 14.99 | 34.72 | >2.5 |
| **28** | g•u•guGcACUucgcuuc acaD | **34** | u•G•uga(Agn)gCGargu GcAcac•u•u | 30.72 | 52.56 | >2.5 |
| * The test samples were conjugates of double-stranded siRNA analogues. | | | | | | |

**Example 4: Anti-Hepatitis B Virus Activity and Safety Research in Recombinant 8-Type Adeno-Associated Virus Vector-Mediated Hepatitis B Virus Mouse Model (AAV-HBV)**

Experimental objective:

The AAV vector-mediated HBV transfected mouse model is a rapid and efficient HBV model. By utilizing the high hepatotropism of the AAV8 vector, the recombinant 8-type adeno-associated virus carrying 1.3 copies of HBV genome (rAAV8-1.3HBV) is injected via tail vein of mice, which can efficiently introduce the carried 1.3 copies of HBV genome into liver cells. Due to the characteristics of AAV viral vector, the vector mediated by it can express continuously for a long period of time, and HBV DNA can be continuously replicated and HBsAg and HBeAg can be expressed in the liver of mice by applying the AAV/HBV model.

By using the AAV/HBV mouse model, HBsAg, HBeAg, DNA and pgRNA in the serum of mice and the weight of mice were detected after treating the mice with the test compound, thus evaluating the *in vivo* anti-HBV effect and safety of the test compound.

Experimental materials:

C57BL/6 mice, PBS (RNase free) as vehicle, test compounds, recombinant virus rAAV8-1.3HBV.

Main reagents of the project include QIAamp96 DNA Kit (Qiagen, 51162), FastStart Universal Probe Master (Rox) (Roche, 04914058001), HBsAg assay kit (Autobio-CL0310); HBeAg assay kit (Autobio-CL0918), PureLink™ Pro 96 Viral RNA/DNA kit (Invitrogen, 12280-096A) and

FastQuant RT Kit (with gDNase) (TIANGEN, KR106-02). Main instruments include centrifuge (Beckman Allegra X-15R), multifunctional microplate reader (BioTek, Synergy 2), fluorescent quantitative PCR instrument (Applied Biosystems, 7900HT Fast Real-time PCR system) and microplate reader (Molecular Devices, SpectraMax 340PC384).

**Experimental method:**

a) Mice were subjected to subcutaneous injection on day 34 after virus injection, and this day was set as day 0. Before administration, all the mice were subjected to submaxillary blood sampling for plasma collection. The specific administration regimen is shown in Table 3.

b) The mice were subjected to blood sampling via submaxillary vein on days 0, 14, 21, 28 and 32 after administration for plasma collection, and the blood samples were anticoagulated with $K_2$-EDTA and centrifuged at 4 °C and 7000 g/min for 10 min to collect plasma. The specific time for blood sampling is shown in Table 3.

c) On day 35 or 42, all the mice were subjected to blood sampling via submaxillary vein for plasma collection, after which the mice were euthanized by $CO_2$ inhalation. Plasma samples were collected by blood sampling from the heart, and liver samples were collected.

d) The plasma samples were sent for detection.

**Table 3. Scheme for *in vivo* experiment**

| Number of mice | Administration design | | | | Non-endpoint blood sampling scheme | Endpoint of experiment |
|---|---|---|---|---|---|---|
| | Test compound | Administration amount (mg/kg) | Administration volume (mL/kg) | Administration regimen | | |
| **5** | **Vehicle** | / | **5** | Day 34 after virus injection was set as day 0, and drug administration was performed once via subcutaneous injection on day 0 and day 29. | Day 34 after virus injection was set as day 0, and the blood sampling time was days 0, 7, 14, 21, 28, 32 and 35. | On day 35 after administration, the mice were subjected to blood sampling via submaxillary venous plexus for plasma collection, after which the mice were euthanized by $CO_2$ inhalation. Plasma samples were collected by blood sampling from the heart, and liver samples were collected. |
| **5** | **WRG01***[1] | **3** | | | | |
| **5** | **WR007***[2] | **3** | | Day 34 after virus injection was set as day 0, and on day 0, drug administration was performed once by subcutaneous injection. | | / |
| **5** | **WR012***[3] | **3** | | | | |

*[1]: WRG01 is a conjugate, in which the sense strand is SEQ ID NO: 16, the antisense strand is SEQ ID NO: 23, and the conjugate group is D.
*[2]: WR007 is a conjugate, in which the sense strand is SEQ ID NO: 42, the antisense strand is SEQ ID NO: 23, and the conjugate group is D.
*[3]: WR012 is a conjugate, in which the sense strand is SEQ ID NO: 16, the antisense strand is SEQ ID NO: 47, and the conjugate group is D.
/: the endpoint has not been reached.

**Sample analysis:**

ELISA assay for the content of HBsAg and HBeAg in the serum of mice: reference was made to the instructions of the HBsAg ELISA kit (Autobio, CL 0310) and HBeAg ELISA kit (Autobio, CL0918) for experimental procedures.

qPCR assay for the content of HBV DNA in the plasma of mice: HBV DNA in plasma was extracted, and reference was made to the instructions of QIAamp 96 DNA Blood Kit for experimental procedures, thus detecting the content of HBV DNA in the plasma of mice by qPCR.

RT-qPCR assay for the content of HBV pgRNA in the plasma of mice: HBV pgRNA was extracted from plasma and reference was made to the instructions of PureLink™ Pro 96 Viral RNA/DNAKit for experimental procedures. The DNA was digested and the RNA was reverse transcribed into cDNA using a 3'RACE primer containing hepatitis B virus specific sequence, and reference was made to the instructions of FastQuant RT Kit (with gDNase) for experimental procedures. Finally, the content of cDNA was quantitatively detected by qPCR, namely detecting the content of HBV pgRNA in the plasma of mice.

Mean$\pm$standard error of mean was used to express the value of each group of mouse samples, and n = 5 unless otherwise specified. Statistical analysis was performed using Student's *t*-test.

**Experimental results:**

a) The anti-HBV activity of the test compounds in AAV/HBV mouse models was evaluated according to the content of HBsAg in serum. The results are shown in Table 4, Table 4-1, FIG. 1 and FIG. 6. The content of HBsAg in the plasma of mice was determined by ELISA. Error bars represent the standard error. Day 0: all mice were subjected to administration of vehicle or compound for the first time. Day 29: the mice in the experimental group WRG01 and mice in the corresponding blank control group were inoculated with vehicle or compound for the second time.

**Table 4. $Log_{10}$ [HBsAg (IU/mL)] of mice on different days after administration**

| Days of detection (day) | Blank (SC) | WRG01 (SC) |
|---|---|---|
| 0 | 4.70 | 4.72 |
| 7 | 4.82 | 2.90 |
| 14 | 4.43 | 2.90 |
| 21 | 4.94 | 3.28 |
| 28 | 4.84 | 3.77 |
| 35 | 4.78 | 2.83 |

**Table 4-1. $Log_{10}$ [HBsAg (IU/mL)] of mice on different days after administration**

| Days of detection (day) | Blank (SC) | WR007(SC) | WR012 (SC) |
|---|---|---|---|
| 0 | 4.58 | 4.19 | 4.47 |
| 7 | 4.15 | 1.92 | 2.00 |
| 14 | 4.57 | 2.29 | 2.20 |
| 21 | 4.41 | 2.63 | 2.36 |
| 28 | 4.76 | 2.94 | 3.10 |
| 35 | 4.62 | 3.31 | 3.19 |

b) The anti-HBV activity of the test compounds in AAV/HBV mouse models was evaluated according to the content of HBeAg in serum. The results are shown in Table 5, Table 5-1, FIG. 2 and FIG. 7. The content of HBeAg in the plasma of mice was determined by ELISA. Error bars represent the standard error. Day 0: all mice were subjected to administration of vehicle or compound for the first time.

**Table 5. $Log_{10}$ [HBeAg (PEIU/mL)] of mice on different days after administration**

| Days of detection (day) | Blank (SC) | WRG01 (SC) |
|---|---|---|
| 0 | 3.56 | 3.51 |
| 7 | 3.37 | 2.89 |
| 14 | 3.56 | 3.06 |
| 21 | 3.66 | 3.22 |

**Table 5-1. $Log_{10}$ [HBeAg (PEIU/mL)] of mice on different days after administration**

| Days of detection (day) | Blank (SC) | WR007 (SC) | WR012 (SC) |
|---|---|---|---|
| 0 | 3.44 | 3.35 | 3.40 |
| 7 | 3.24 | 2.49 | 2.53 |
| 14 | 3.57 | 2.80 | 2.89 |

(continued)

| Days of detection (day) | Blank (SC) | WR007 (SC) | WR012 (SC) |
|---|---|---|---|
| 21 | 3.32 | 2.81 | 2.82 |
| 28 | 3.38 | 2.95 | 2.91 |
| 35 | 3.37 | 3.09 | 3.02 |

c) The anti-HBV activity of the test compounds in AAV/HBV mouse models was evaluated according to the content of DNA in serum. The results are shown in Table 6, Table 6-1, FIG. 3 and FIG. 8. The content of HBV DNA in the plasma of mice was determined by quantitative PCR. Error bars represent the standard error. Day 0: all mice were subjected to administration of vehicle or compound for the first time. Day 29: all the mice were inoculated with vehicle or compound for the second time.

**Table 6. $Log_{10}$ [DNA (copy number/μL)] of mice on different days after administration**

| Days of detection (day) | Blank (SC) | WRG01 (SC) |
|---|---|---|
| 0 | 5.27 | 4.84 |
| 7 | 5.39 | 3.93 |
| 14 | 5.51 | 3.97 |
| 21 | 5.63 | 4.37 |

**Table 6-1. $Log_{10}$ [DNA (copy number/μL)] of mice on different days after administration**

| Days of detection (day) | Blank (SC) | WR007 (SC) | WR012 (SC) |
|---|---|---|---|
| 0 | 5.53 | / | / |
| 7 | 4.98 | / | / |
| 14 | 5.34 | 3.44 | 3.95 |
| 21 | 5.45 | 3.71 | 4.21 |
| 28 | 5.63 | 4.08 | 4.66 |
| 35 | 5.26 | 4.42 | 4.78 |
| /: no data were obtained. | | | |

d) The anti-HBV activity of the test compounds in AAV/HBV mouse models was evaluated according to the content of pgRNA in serum. The results are shown in Table 7 and FIG. 4. The content of HBV pgRNA in the plasma of mice was determined by quantitative PCR. Error bars represent the standard error. Day 0: all mice were subjected to administration of vehicle or compound for the first time. Day 29: all the mice were inoculated with vehicle or compound for the second time.

**Table 7. $Log_{10}$ [pgRNA (copy number/μL)] of mice on different days after administration**

| Days of detection (day) | Blank (SC) | WRG01 (SC) |
|---|---|---|
| 0 | 4.92 | 4.56 |
| 7 | 4.96 | 3.28 |
| 14 | 4.93 | 3.26 |
| 21 | 5.02 | 3.50 |
| 28 | 5.06 | 4.13 |
| 35 | 5.17 | 3.37 |

e) The change in body weight is shown in FIG. 5. The comparison was performed with the body weight on day 0 used

as a baseline. As per IACUC regulation, losing of 20% body weight is considered as a humane endpoint, and any mouse that loses more than 20% of its body weight should be removed from the experiment. None of the mice in this experiment was removed due to weight loss.

**Experimental conclusion:**

In this experiment, the test compounds were able to significantly reduce HBsAg, DNA and pgRNA in AAV/HBV mouse models. Meanwhile, the test compounds also had a certain inhibiting effect on HBeAg. During the treatment with the test compounds, the mice showed good tolerance and the body weight gradually increased.

**Example 5: HBV *In Vitro* Assay of HepG2.2.15 Cells**

**1. Experimental objective:**

The content of HBV DNA in the HepG2.2.15 cell culture supernatant was detected using real-time qPCR, and the content of HBsAg and HBeAg was detected using ELISA; the content of HBV RNA in cells was detected using qRT-PCR, the $EC_{50}$ value of the compound was used as an index to evaluate the inhibitory effect of the compound on HBV, and the influence of the test compound on the cell viability was detected using the CCK8 method.

**2. Experimental materials:**

2.4. Cell line: HepG2.2.15 cells

HepG2.2.15 cell culture medium (DMEM/F12, Invitrogen-11330032; 10% serum, Hyclone-SV30087.0; 100 units/mL penicillin and 100 μg/mL streptomycin, Hyclone-SV30010; 1% non-essential amino acids, Invitrogen-11140050; 2 mM L-glutamine, Invitrogen-25030081; 300 μg/mL Geneticin, Invitrogen-10131027).

2.5. Reagents

Opti-MEM (Gibco-31985-070); Lipofectamine® RNAiMAX (Invitrogen-13778-150); CCK8 (Life-iLab-AC11L057); high-throughput DNA purification kit (QIAamp 96 DNA Blood Kit, Qiagen-51162); RNA preparation RNEASY Kit (RNeasy 96 Kit (12), Qiagen-74182); quantitative fast start universal probe reagent (FastStart Universal Probe Master, Roche-04914058001); FastKing cDNA first strand synthesis kit (TianGen-KR106-02); HBsAg quantitative assay kit (Autobio-CL 0310); HBeAg quantitative assay kit (Autobio-CL 0312).

2.6. Consumables and instrument:

Collagen I 96 Well White/Clear Flat Bottom TC-Treated Microplate (Corning BioCoat-356650); $CO_2$ incubator (HERA-CELL-240); fluorescent quantitative PCR instrument (Applied Biosystems-7900 real time PCR system); fluorescent quantitative PCR instrument (Applied Biosystems-QuantStudio 6 Flex); microplate reader (Molecular Device-SpectraMax M2e); microplate reader (BioTek-Synergy 2).

**3. Experimental procedures and method:**

3.1. On day one, transfection of siRNA and cell plating were performed simultaneously, and the brief procedures are as follows: HepG2.2.15 cells were washed with DPBS and digested with 0.05% trypsin, and then the digestion was terminated with DMEM/F12 medium containing 10% FBS; the cells were then centrifuged, resuspended, gently pipetted into single cells and counted. The volume of desired transfection reagent was set according to certain ratio (Table 8), and the cells were incubated for 15 min at room temperature.

**Table 8. Allocation of Lipofectamine® RNAiMAX**

| Reagent | Ratio (allocation for one well as an example) |
|---|---|
| Lipofectamine® RNAiMAX | 1.5 |
| Opti-MEM | 23.5 |

The siRNA was subjected to 3-fold gradient dilution to get 8 concentrations, and two duplicate wells were set.

15 μL of RNAiMAX/Opti-MEM mixture was well mixed with 15 μL of siRNA at different concentrations, and the mixture was incubated for 15 min at room temperature. 10 μL of the above mixed solution was added into a 96-well cell culture plate, then 90 μL of cell suspension was added, and the final cell density was 15,000 cells/well and the final volume was 100 μL/well. The cells were then incubated in an incubator at 37 °C and 5% $CO_2$.

3.2. On day four, the original culture medium was replaced with a fresh culture medium containing the compound, and the transfection procedure was the same as that of day one.

3.3. On day seven, the culture solution in the culture well was collected and sampling was performed. A part of the samples were used for ELISA assay of the content of HBsAg and HBeAg; a part of the samples were used for DNA extraction by using a high-throughput DNA purification kit (Qiagen-51162); after the supernatant was collected, the cell viability was detected according to instructions of the CCK-8 kit, and the absorbance (450 nm/650 nm) of each well was detected with a microplate reader (SpectraMax M2e); HBV RNA was extracted from the cell culture using the RNeasy 96 kit extraction kit (Qiagen-74182) with reference to the kit instructions.

3.4. The preparation of the PCR reaction solution is shown in Table 9:

**Table 9. Preparation of PCR reaction solution**

| Items | Volume required for 1 well (μL) | Volume required for 100 wells (μL) |
|---|---|---|
| Quantitative fast start universal probe reagent | 5 | 500 |
| Forward primer (10 μmol) | 0.4 | 40 |
| Reverse primer (10 μmol) | 0.4 | 40 |
| Probe (10 μmol) | 0.2 | 20 |
| AE | 2 | 200 |

8 μL of the reaction mixture was added into each well of the 96-well PCR plate, and then 2 μL of sample DNA or HBV DNA standard substance was added into each well.

The reaction conditions of PCR are as follows: heating for 10 min at 95 °C, then denaturing for 15 s at 95 °C and extending for 1 min at 60 °C, 40 cycles in total.

3.5. Reference was made to the instructions of the product for specific procedures of ELISA assay for content of HBsAg and HBeAg, and the brief procedures are as follows: 50 μL of sample and 50 μL of standard substance were each added into a reaction plate, then enzyme conjugate was added at 50 μL/well, and the mixture was well mixed by shaking and incubated at 37 °C for 60 min in a warm bath; the plate was washed 5 times by using a washing solution, luminescent substrate was then added at 50 μL/well, and the mixture was well mixed and reacted at room temperature in the dark for 10 min, and finally the chemiluminescence intensity was detected by using a microplate reader.

3.6. The HBV RNA in cell culture was extracted by using the RNeasy 96 kit extraction kit (Qiagen, 74182) with reference to the kit instructions. Cells were lysed with 150 μL of RLT, and finally RNA was eluted with 50 μL of RNase-free water. A random primer was added according to the instructions of the reverse transcription kit (Tiangen, KR106) for reverse transcription into cDNA, then an HBV specific primer was used for detecting total RNA in the sample; meanwhile, GAPDH primers and probes were used for specifically detecting GAPDH cDNA, and the qPCR method was used for quantifying the HBV cDNA in the sample.

qPCR reaction: 95 °C, 10 min; 95 °C, 15 s; 60 °C, 1 min, 40 cycles in total. The content of HBV RNA in each sample was calculated according to the Ct value of the sample.

The expression level of HBV mRNA, the target gene of each sample, was calculated by the relative quantification method of ΔΔCt. The relative expression level of the target gene was expressed by 2-ΔΔCT, and the calculation formula is as follows:

$$\Delta CT = \text{mean Ct value of target gene} - \text{mean Ct value of reference gene};$$

$$\Delta\Delta CT = \Delta CT\ (\text{treatment group}) - \Delta CT\ (\text{RNAiMAX control group});$$

$$\text{Relative expression level of HBV mRNA} = 2\text{-}\Delta\Delta CT$$

3.7. Data analysis:

Calculation of percentage inhibition:

$$\% \text{ Inh.} = (1 - \text{value in sample/PBS control value}) \times 100.$$

Cell viability% = (detection value of sample – background average detection value of culture solution)/(average detection value of control group – background average detection value of culture solution) ×100

Calculation of $EC_{50}$ and $CC_{50}$: the 50% inhibitory concentration ($EC_{50}$) of the compound for HBV and the drug concentration at 50% cell death ($CC_{50}$) were calculated using GraphPad Prism software.

**Table 10. Results of test sequences in reducing HBsAg, HBeAg, DNA and RNA levels in cells**

| Test sequences | | | | Experimental results | | | | |
|---|---|---|---|---|---|---|---|---|
| SEQ ID NO | Sequence of sense strand (5'-3') | SEQ ID NO | Sequence of antisense strand (5'-3') | HBsAg $EC_{50}$ (nM) | HBeAg $EC_{50}$ (nM) | DNA $EC_{50}$ (nM) | RNA $EC_{50}$ (nM) | Cell viability $CC_{50}$ (nM) |
| **16** | g•r•guGcACUuc gcuucacaD | **23** | u•G•ugaArCGaag uGcAcac•u•u | 0.179 | 0.755 | 0.14 | 0.87 | >50 |
| **28** | g•u•guGcACUuc gcuucacaD | **17** | u•G•ugargCGaag uGcAcac•u•u | 0.105 | 0.37 | 0.16 | 0.567 | >50 |
| **28** | g•u•guGcACUuc gcuucacaD | **23** | u•G•ugaArCGaag uGcAcac•u•u | 0.079 | 0.587 | 0.135 | 3.197 | >50 |
| **28** | g•u•guGcACUuc gcuucacaD | **34** | u•G•uga(Agn)gC GarguGcAcac•u• u | 0.263 | 1.268 | 0.553 | 0.963 | >50 |
| **42** | g•r•guGcACUuc gcurcacaD | **23** | u•G•ugaArCGaag uGcAcac•u•u | 0.07 | 0.389 | 0.019 | 0.65 | >50 |
| **43** | g•r•guGcACUucr cuucacaD | **23** | u•G•ugaArCGaag uGcAcac•u•u | 0.231 | / | / | / | >50 |
| **16** | g•r•guGcACUuc gcuucacaD | **44** | u•G•uraArCGaag uGcAcac•u•u | 0.072 | 2.202 | 0.06 | / | >50 |
| **16** | g•r•guGcACUuc gcuucacaD | **45** | u•G•uga(Agn)rC GaaguGcAcac•u• u | 0.054 | 3.707 | 0.15 | / | >50 |
| **16** | g•r•guGcACUuc gcuucacaD | **46** | u•G•uga(Agn)gC GaaruGcAcac•u• u | 0.497 | / | / | / | >50 |
| **16** | g•r•guGcACUuc gcuucacaD | **47** | VPu•G•ugaArCG aaguGcAcac•u•u | 0.082 | 0.205 | 0.037 | 0.68 | >50 |
| **16** | g•r•guGcACUuc gcuucacaD | **48** | VPu•G•uga(Agn) rCGaaguGcAcac• u•u | 0.104 | 3.83 | 0.105 | / | >50 |
| /: no data were obtained.<br>* The test samples were conjugates of double-stranded siRNA analogues. | | | | | | | | |

**Example 6: Dose Exploration for Effective Anti-Hepatitis B Virus Activity in AAV-HBV Mouse Models**

By using the AAV/HBV mouse models, HBsAg in the serum of mice was detected after treating the mice with the test compound at different doses, thus evaluating the *in vivo* anti-HBV effect of the test compound.

Experimental materials:

C57BL/6 mice, PBS (RNase free) as vehicle, test compounds, recombinant virus rAAV8-1.3HBV. Main reagents of the project include FastStart Universal Probe Master (Rox) (Roche, 04914058001) and HBsAg assay kit (Autobio, CL0310). Main instruments include centrifuge (Beckman Allegra X-15R), multifunctional microplate reader (BioTek, Synergy 2) and microplate reader (Molecular Devices, SpectraMax 340PC384).

Experimental method:

a) All the mice were subjected to subcutaneous injection on day 34 after virus injection, and this day was set as day 0. Before administration, all the mice were subjected to submaxillary blood sampling for plasma collection. Drug administration was performed once on day 0. The specific administration regimen is shown in Table 14.

b) All the mice were subjected to blood sampling via submaxillary vein on days 0, 14, 21, 28 and 35 after administration for plasma collection, and the blood samples were anticoagulated with $K_2$-EDTA and centrifuged at 4 °C and 7000 g/min for 10 min to collect plasma. The specific time for blood sampling is shown in Table 11.

c) On day 42, all the mice were subjected to blood sampling via submaxillary vein for plasma collection, after which the mice were euthanized by $CO_2$ inhalation. Plasma samples were collected by blood sampling from the heart, and liver samples were collected.

d) All the plasma samples were sent for detection.

**Table 11. Scheme for *in two* experiment**

<table>
<tr><th rowspan="2">Number of mice</th><th colspan="4">Administration design</th><th rowspan="2">Non-endpoint blood sampling scheme</th><th rowspan="2">Endpoint of experiment</th></tr>
<tr><th>Test compound</th><th>Administration amount (mg/kg)</th><th>Administration volume (mL/kg)</th><th>Administration regimen</th></tr>
<tr><td>5</td><td>Vehicle</td><td>/</td><td rowspan="5">5</td><td rowspan="5">Day 34 after virus injection was set as day 0, and on day 0, drug administration was performed once by subcutaneous injection.</td><td rowspan="5">Day 34 after virus injection was set as day 0, and the blood sampling time was days 0, 7, 14, 21, 28 and 35.</td><td></td></tr>
<tr><td>5</td><td rowspan="4">WRG01*</td><td>0.3</td><td rowspan="2">On day 42 after administration, the mice were subjected to blood sampling via submaxillary venous plexus for plasma collection, after which the mice were euthanized by $CO_2$ inhalation. Plasma samples were collected by blood sampling from the heart, and liver samples were collected.</td></tr>
<tr><td>5</td><td>1</td></tr>
<tr><td>5</td><td>3</td><td>/</td></tr>
<tr><td>5</td><td>10</td><td>/</td></tr>
<tr><td colspan="7">*: WRG01 is a conjugate, in which the sense strand is SEQ ID NO: 16, the antisense strand is SEQ ID NO: 23, and the conjugate group is D.<br>/: the endpoint has not been reached.</td></tr>
</table>

**Sample analysis:**

ELISA assay for the content of HBsAg in the serum of mice: reference was made to the instructions of the HBsAg ELISA kit (Autobio, CL 0310) for experimental procedures.

Mean±standard error of mean was used to express the value of each group of mouse samples, and n = 5 unless otherwise specified. Statistical analysis was performed using Student's t-test.

**Experimental results:**

The anti-HBV activity of the test compound in AAV/HBV mouse models was evaluated by detecting the content of HBsAg in serum. The results are shown in Table 12 and FIG. 9. The content of HBsAg in the plasma of mice was determined by ELISA. Error bars represent the standard error.

Day 0: all mice were subjected to administration of vehicle or compound for the first time.

**Table 12. $Log_{10}$ [HBsAg (IU/mL)] of mice on different days after administration**

| Days of detection (day) | Blank (SC) | WRG01, 0.3 mpk (SC) | WRG01, 1 mpk (SC) | WRG01, 3 mpk (SC) | WRG01,10 mpk (SC) |
|---|---|---|---|---|---|
| 0 | 4.58 | 4.49 | 4.53 | 4.31 | 4.56 |
| 7 | 4.15 | 3.67 | 2.93 | 2.19 | 1.98 |
| 14 | 4.57 | 4.18 | 3.60 | 2.26 | 2.12 |
| 21 | 4.41 | 4.46 | 3.80 | 2.48 | 2.17 |
| 28 | 4.76 | 4.76 | 4.25 | 3.22 | 2.99 |
| 35 | 4.62 | 4.65 | 4.31 | 3.40 | 3.12 |

**Experimental conclusion:**

In this experiment, the test compound WRG01 exhibited good dose dependence for reducing HBsAg in AAV/HBV mouse models; that is, its activity for reducing HBsAg increased along with the increase in the drug dose, and it exhibited long-term efficacy in inhibiting HBsAg.

**Example 7: Drug Concentration Test in Mouse Plasma, Liver and Kidney**

In this study, C57BL/6 mice were subjected to a single administration via subcutaneous injection, plasma and tissue samples were collected at various time points after drug administration, and metabolic levels of the compound in the mice were evaluated by SL-qPCR detection for siRNA levels in plasma and tissues.

**Table 13. Scheme for *in vivo* experiment**

| Number of mice | Administration design | | | | Non-endpoint peripheral blood collection scheme | Endpoint of experiment |
|---|---|---|---|---|---|---|
| | Test compound | Administration amount (mg/kg) | Administration volume (mL/kg) | Administration regimen | | |
| 3 | WRG01* | 3 | 5 | Drug administration was performed once via subcutaneous injection on day 0. | The blood sampling time was 0.083 h after administration. | At 0.5 h after administration, the mice were subjected to blood sampling via submaxillary venous plexus for plasma collection, after which the mice were euthanized by $CO_2$ inhalation, and liver and kidney samples were collected. |
| 3 | | 3 | | | The blood sampling time was 0.25 h after administration. | At 1 h after administration, the mice were subjected to blood sampling via submaxillary venous plexus for plasma collection, after which the mice were euthanized by $CO_2$ inhalation, and liver and kidney samples were collected. |
| 3 | | 3 | | | / | At 2 h after administration, the mice were subjected to blood sampling via submaxillary |

(continued)

| Number of mice | Administration design | | | | Non-endpoint peripheral blood collection scheme | Endpoint of experiment |
|---|---|---|---|---|---|---|
| | Test compound | Administration amount (mg/kg) | Administration volume (mL/kg) | Administration regimen | | |
| | | | | | | venous plexus for plasma collection, after which the mice were euthanized by $CO_2$ inhalation, and liver and kidney samples were collected. |
| 3 | | 3 | | | / | At 4 h after administration, the mice were subjected to blood sampling via submaxillary venous plexus for plasma collection, after which the mice were euthanized by $CO_2$ inhalation, and liver and kidney samples were collected. |
| 3 | | 3 | | | / | At 8 h after administration, the mice were subjected to blood sampling via submaxillary venous plexus for plasma collection, after which the mice were euthanized by $CO_2$ inhalation, and liver and kidney samples were collected. |

(continued)

| Number of mice | Administration design | | | | Non-endpoint peripheral blood collection scheme | Endpoint of experiment |
|---|---|---|---|---|---|---|
| | Test compound | Administration amount (mg/kg) | Administration volume (mL/kg) | Administration regimen | | |
| 3 | | 3 | | | / | At 32 h after administration, the mice were subjected to blood sampling via submaxillary venous plexus for plasma collection, after which the mice were euthanized by $CO_2$ inhalation, and liver and kidney samples were collected. |
| 3 | | 3 | | | The blood sampling time was 48 h and 96 h after administration. | At 168 h after administration, the mice were subjected to blood sampling via submaxillary venous plexus for plasma collection, after which the mice were euthanized by $CO_2$ inhalation, and liver and kidney samples were collected. |
| *: WRG01 is a conjugate, in which the sense strand is SEQ ID NO: 16, the antisense strand is SEQ ID NO: 23, and the conjugate group is D.<br>/: blood sampling was not performed at "non-endpoint" time, only at endpoint. | | | | | | |

**Experimental results:**

The siRNA levels in plasma, liver and kidney of mice at different time points after administration were detected using the SL-qPCR method (reference: Nair et al., Nucleic Acids Research (2017), 45, 10969-10977) and the results are shown in FIG. 10.

**Experimental conclusion:**

In this experiment, the test compound WRG01 had good tissue distribution and metabolic stability in the C57BL/6 mouse models. WR-G01 has large liver exposure, long half-life period and liver-to-blood ratio of more than 500 times, which proves WRG01 has metabolic stability and high liver-targeting property.

### Example 8: Blood Biochemical Test in Mice with FRG-KO Humanized Liver

The humanized FRG mouse is one of the most commonly used humanized liver models, usually with a humanization rate as high as 70%. Because human liver cells are planted in the liver of the mouse, the natural HBV infection and cccDNA replication process of a human body can be better simulated, and meanwhile, this model can well predict the pharmacokinetics and hepatotoxicity of the human body.

In this study, the humanized FRG mice were subjected to multiple times of drug administration, plasma samples at different time points after administration were collected, and the toxic and side effect of the compound on the liver of the mice was evaluated by detecting ALT, AST and bilirubin levels in the plasma. In this experiment, the test compound did not cause significant inflammatory response of the humanized liver, indicating good safety in human body.

**Table 14. Scheme for *in vivo* experiment**

| Number of mice | Administration design | | | | Non-endpoint peripheral blood collection scheme | Endpoint of experiment |
|---|---|---|---|---|---|---|
| | Test compound | Administration amount (mg/kg) | Administration volume (mL/kg) | Administration regimen | | |
| **3** | **Vehicle** | / | **5** | Drug administration was performed once via subcutaneous injection on days 0, 21, 28, 35 and 42. | The blood sampling time was 1 day before administration, and days 7, 14, 21, 28, 35 and 42 after administration. | / |
| **3** | **WRG01*** | **15** | | | | |
| **3** | | **50** | | | | |
| *: WRG01 is a conjugate, in which the sense strand is SEQ ID NO: 16, the antisense strand is SEQ ID NO: 23, and the conjugate group is D.<br>/: the endpoint has not been reached. | | | | | | |

The present disclosure exhibits unpredictably excellent inhibitory activity on HBsAg and HBeAg while effectively inhibiting expression of HBV DNA and pgRNA, which demonstrates that the present disclosure can inhibit the activity of hepatitis B virus. Meanwhile, the present disclosure has good tissue distribution and metabolic stability, and it has high liver-targeting property and is expected to have little effect on mouse liver function. It will provide an efficient treatment means for hepatitis B in clinic, such as chronic hepatitis B.

## Claims

1. A double-stranded siRNA analogue, a conjugate thereof or a salt thereof, comprising a sense strand and an antisense strand, wherein the antisense strand comprises a sequence obtained by replacing one or more nucleotide residues in a sequence set forth in SEQ ID NO: 2 with r, and the r is

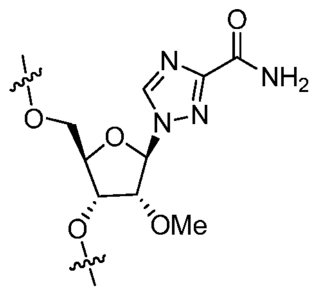

**r** ,

wherein each of nucleotides and r in the siRNA analogue is independently modified or unmodified.

2. The double-stranded siRNA analogue, the conjugate thereof or the salt thereof according to claim 1, wherein 70%, 75%, 80%, 85%, 90% or 95% or more of the nucleotides and r in the double-stranded siRNA analogue are modified; optionally, all the nucleotides and r in the double-stranded siRNA analogue are modified.

3. The double-stranded siRNA analogue, the conjugate thereof or the salt thereof according to claim 1 or 2, wherein the modification comprises methoxy modification, fluoro modification, phosphorothioate linkage, replacement of a nucleotide with (S)-glycerol nucleic acid or replacement of a nucleotide with (E)-vinyl phosphate.

4. The double-stranded siRNA analogue, the conjugate thereof or the salt thereof according to any one of claims 1-3, wherein the antisense strand comprises a sequence obtained by replacing one, two, three, four or five nucleotide residues in the sequence set forth in SEQ ID NO: 2 with r; optionally, the antisense strand comprises a sequence obtained by replacing one nucleotide residue in the sequence set forth in SEQ ID NO: 2 with r.

5. The double-stranded siRNA analogue, the conjugate thereof or the salt thereof according to any one of claims 1-4, wherein the r replacement occurs at any position of the SEQ ID NO: 2.

6. The double-stranded siRNA analogue, the conjugate thereof or the salt thereof according to any one of claims 1-5, wherein the SEQ ID NO: 2 optionally comprises an overhang at the 5' end and/or 3' end; optionally, the SEQ ID NO: 2 comprises an overhang of 0, 1, 2, 3, 4 or 5 nucleotides at the 5' end and/or 3' end; optionally, the SEQ ID NO: 2 comprises an overhang at the 3' end, and the overhang is selected from modified or unmodified UU.

7. The double-stranded siRNA analogue, the conjugate thereof or the salt thereof according to any one of claims 1-6, wherein the antisense strand comprises or consists of a sequence set forth in SEQ ID NO: 4 or SEQ ID NO: 17, SEQ ID NO: 6 or SEQ ID NO: 19, SEQ ID NO: 7 or SEQ ID NO: 20, SEQ ID NO: 8 or SEQ ID NO: 21, SEQ ID NO: 9 or SEQ ID NO: 22, SEQ ID NO: 10 or SEQ ID NO: 23, SEQ ID NO: 11 or SEQ ID NO: 24, SEQ ID NO: 29 or SEQ ID NO: 33, SEQ ID NO: 30 or SEQ ID NO: 34, SEQ ID NO: 31 or SEQ ID NO: 35, SEQ ID NO: 32 or SEQ ID NO: 36, SEQ ID NO: 39 or SEQ ID NO: 44, SEQ ID NO: 10 or SEQ ID NO: 45, SEQ ID NO: 40 or SEQ ID NO: 46, SEQ ID NO: 10 or SEQ ID NO: 47, or SEQ ID NO: 10 or SEQ ID NO: 48.

8. The double-stranded siRNA analogue, the conjugate thereof or the salt thereof according to any one of claims 1-7, wherein the sense strand comprises or consists of a sequence set forth in SEQ ID NO: 1 or SEQ ID NO: 28.

9. The double-stranded siRNA analogue, the conjugate thereof or the salt thereof according to any one of claims 1-8, wherein the sense strand comprises a sequence obtained by replacing one or more nucleotide residues in the sequence set forth in the SEQ ID NO: 1 with r; optionally, the sense strand comprises a sequence obtained by replacing one, two, three, four or five nucleotide residues in the sequence set forth in the SEQ ID NO: 1 with r.

10. The double-stranded siRNA analogue, the conjugate thereof or the salt thereof according to any one of claims 1-9, wherein the r replacement occurs at positions 1-19 of the 5' end of the SEQ ID NO: 1.

11. The double-stranded siRNA analogue, the conjugate thereof or the salt thereof according to any one of claims 1-10, wherein the sequence of the sense strand comprises or consists of a sequence set forth in SEQ ID NO: 5 or SEQ ID NO: 18, SEQ ID NO: 3 or SEQ ID NO: 16, SEQ ID NO: 14 or SEQ ID NO: 27, SEQ ID NO: 13 or SEQ ID NO: 26, SEQ ID NO: 12 or SEQ ID NO: 25, SEQ ID NO: 37 or SEQ ID NO: 42, or SEQ ID NO: 38 or SEQ ID NO: 43.

12. The double-stranded siRNA analogue, the conjugate thereof or the salt thereof according to any one of claims 1-11, wherein the double-stranded siRNA analogue is any one of S18-S28:

S18: the sense strand is SEQ ID NO: 1 or SEQ ID NO: 28, and the antisense strand is SEQ ID NO: 4 or SEQ ID NO: 17,
S19: the sense strand is SEQ ID NO: 1 or SEQ ID NO: 28, and the antisense strand is SEQ ID NO: 6 or SEQ ID NO: 19,
S20: the sense strand is SEQ ID NO: 1 or SEQ ID NO: 28, and the antisense strand is SEQ ID NO: 7 or SEQ ID NO: 20,
S21: the sense strand is SEQ ID NO: 1 or SEQ ID NO: 28, and the antisense strand is SEQ ID NO: 8 or SEQ ID NO: 21,
S22: the sense strand is SEQ ID NO: 1 or SEQ ID NO: 28, and the antisense strand is SEQ ID NO: 9 or SEQ ID NO: 22,

S23: the sense strand is SEQ ID NO: 1 or SEQ ID NO: 28, and the antisense strand is SEQ ID NO: 10 or SEQ ID NO: 23,
S24: the sense strand is SEQ ID NO: 1 or SEQ ID NO: 28, and the antisense strand is SEQ ID NO: 11 or SEQ ID NO: 24,
S25: the sense strand is SEQ ID NO: 1 or SEQ ID NO: 28, and the antisense strand is SEQ ID NO: 29 or SEQ ID NO: 33,
S26: the sense strand is SEQ ID NO: 1 or SEQ ID NO: 28, and the antisense strand is SEQ ID NO: 30 or SEQ ID NO: 34,
S27: the sense strand is SEQ ID NO: 1 or SEQ ID NO: 28, and the antisense strand is SEQ ID NO: 31 or SEQ ID NO: 35, and
S28: the sense strand is SEQ ID NO: 1 or SEQ ID NO: 28, and the antisense strand is SEQ ID NO: 32 or SEQ ID NO: 36, or,

wherein the double-stranded siRNA analogue is any one of S1-S17:

S1: the sense strand is SEQ ID NO: 3 or SEQ ID NO: 16, and the antisense strand is SEQ ID NO: 4 or SEQ ID NO: 17,
S2: the sense strand is SEQ ID NO: 5 or SEQ ID NO: 18, and the antisense strand is SEQ ID NO: 4 or SEQ ID NO: 17,
S3: the sense strand is SEQ ID NO: 3 or SEQ ID NO: 16, and the antisense strand is SEQ ID NO: 6 or SEQ ID NO: 19,
S4: the sense strand is SEQ ID NO: 5 or SEQ ID NO: 18, and the antisense strand is SEQ ID NO: 6 or SEQ ID NO: 19,
S5: the sense strand is SEQ ID NO: 3 or SEQ ID NO: 16, and the antisense strand is SEQ ID NO: 7 or SEQ ID NO: 20,
S6: the sense strand is SEQ ID NO: 5 or SEQ ID NO: 18, and the antisense strand is SEQ ID NO: 7 or SEQ ID NO: 20,
S7: the sense strand is SEQ ID NO: 3 or SEQ ID NO: 16, and the antisense strand is SEQ ID NO: 8 or SEQ ID NO: 21,
S8: the sense strand is SEQ ID NO: 5 or SEQ ID NO: 18, and the antisense strand is SEQ ID NO: 8 or SEQ ID NO: 21,
S9: the sense strand is SEQ ID NO: 3 or SEQ ID NO: 16, and the antisense strand is SEQ ID NO: 9 or SEQ ID NO: 22,
S10: the sense strand is SEQ ID NO: 5 or SEQ ID NO: 18, and the antisense strand is SEQ ID NO: 9 or SEQ ID NO: 22,
S11: the sense strand is SEQ ID NO: 3 or SEQ ID NO: 16, and the antisense strand is SEQ ID NO: 10 or SEQ ID NO: 23,
S12: the sense strand is SEQ ID NO: 5 or SEQ ID NO: 18, and the antisense strand is SEQ ID NO: 10 or SEQ ID NO: 23,
S13: the sense strand is SEQ ID NO: 3 or SEQ ID NO: 16, and the antisense strand is SEQ ID NO: 11 or SEQ ID NO: 24,
S14: the sense strand is SEQ ID NO: 5 or SEQ ID NO: 18, and the antisense strand is SEQ ID NO: 11 or SEQ ID NO: 24,
S15: the sense strand is SEQ ID NO: 12 or SEQ ID NO: 25, and the antisense strand is SEQ ID NO: 4 or SEQ ID NO: 17,
S16: the sense strand is SEQ ID NO: 13 or SEQ ID NO: 26, and the antisense strand is SEQ ID NO: 4 or SEQ ID NO: 17, and
S17: the sense strand is SEQ ID NO: 14 or SEQ ID NO: 27, and the antisense strand is SEQ ID NO: 4 or SEQ ID NO: 17,
or,

wherein the double-stranded siRNA analogue is any one of S29-S35:

S29: the sense strand is SEQ ID NO: 37 or SEQ ID NO: 42, and the antisense strand is SEQ ID NO: 10 or SEQ ID NO: 23,
S30: the sense strand is SEQ ID NO: 38 or SEQ ID NO: 43, and the antisense strand is SEQ ID NO: 10 or SEQ ID NO: 23,
S31: the sense strand is SEQ ID NO: 3 or SEQ ID NO: 16, and the antisense strand is SEQ ID NO: 39 or SEQ

ID NO: 44,
S32: the sense strand is SEQ ID NO: 3 or SEQ ID NO: 16, and the antisense strand is SEQ ID NO: 10 or SEQ ID NO: 45,
S33: the sense strand is SEQ ID NO: 3 or SEQ ID NO: 16, and the antisense strand is SEQ ID NO: 40 or SEQ ID NO: 46,
S34: the sense strand is SEQ ID NO: 3 or SEQ ID NO: 16, and the antisense strand is SEQ ID NO: 10 or SEQ ID NO: 47, and
S35: the sense strand is SEQ ID NO: 3 or SEQ ID NO: 16, and the antisense strand is SEQ ID NO: 10 or SEQ ID NO: 48.

**13.** The conjugate of the double-stranded siRNA analogue or the salt thereof according to any one of claims 1-12, wherein the double-stranded siRNA analogue is linked to a pharmaceutically acceptable conjugate group, and the pharmaceutically acceptable conjugate group comprises a GalNAc group; optionally, the pharmaceutically acceptable conjugate group comprises 1 to 5 GalNAc groups.

**14.** The conjugate of the double-stranded siRNA analogue or the salt thereof according to any one of claims 1-13, wherein the double-stranded siRNA analogue is linked to a pharmaceutically acceptable conjugate group, and the pharmaceutically acceptable conjugate group comprises a compound group D

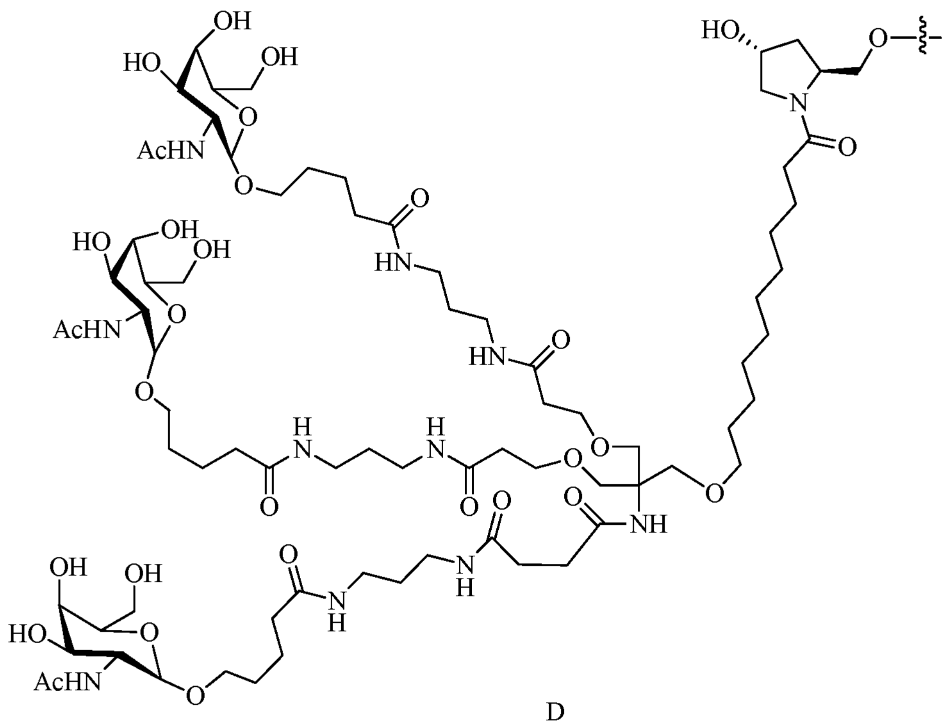

D

.

**15.** The conjugate of the double-stranded siRNA analogue or the salt thereof according to claim 13 or 14, wherein the pharmaceutically acceptable conjugate group is linked to the 3' end of the sense strand of the double-stranded siRNA analogue.

**16.** The double-stranded siRNA analogue, the conjugate thereof or the salt thereof according to any one of claims 1-15, wherein a phosphorothioate moiety of the double-stranded siRNA analogue or the conjugate thereof comprises (R)- and (S)-enantiomers, diastereoisomers, and/or racemic mixtures thereof.

**17.** The double-stranded siRNA analogue, the conjugate thereof or the salt thereof according to any one of claims 1-16, wherein the salt is selected from base addition salts, acid addition salts and combinations thereof; optionally, the base addition salt is selected from sodium, potassium, calcium, ammonium, organic amine, magnesium salts and combinations thereof, and the acid addition salt is selected from salts derived from inorganic acids, salts derived from inorganic acids and combinations thereof; optionally, the inorganic acid is selected from hydrochloric acid, hydrobromic acid, nitric acid, carbonic acid, bicarbonate radical, phosphoric acid, monohydrogen phosphate, dihydrogen phosphate, sulfuric acid, hydrogen sulfate, hydroiodic acid, phosphorous acid and combinations thereof, and the organic acid is selected from acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, methanesulfonic acid and combinations thereof.

**18.** A pharmaceutical composition, comprising the double-stranded siRNA analogue, the conjugate thereof or the salt thereof according to any one of claims 1-17, and optionally a pharmaceutically acceptable carrier or excipient.

**19.** Use of the double-stranded siRNA analogue, the conjugate thereof or the salt thereof according to any one of claims 1-17 or the pharmaceutical composition according to claim 18 for preparing a medicament for the treatment of hepatitis B.

**20.** A method for treating hepatitis B in a subject, comprising the step of administering to the subject the double-stranded siRNA analogue, the conjugate thereof or the salt thereof according to any one of claims 1-17 or the pharmaceutical composition according to claim 18.

## INTERNATIONAL SEARCH REPORT

International application No.
**PCT/CN2021/098682**

**A. CLASSIFICATION OF SUBJECT MATTER**

A61K 31/713(2006.01)i; A61P 31/20(2006.01)i; C12N 15/113(2010.01)i

According to International Patent Classification (IPC) or to both national classification and IPC

**B. FIELDS SEARCHED**

Minimum documentation searched (classification system followed by classification symbols)

A61K A61P C12N

Documentation searched other than minimum documentation to the extent that such documents are included in the fields searched

Electronic data base consulted during the international search (name of data base and, where practicable, search terms used)

CNABS, DWPI, CNTXT, USTXT, EPTXT, WOTXT, JPTXT, CNKI, Web of Science, 万方数据检索系统, pubmed, baidu, patentics, Genbank, EBI, China Patents Biological Sequence Search Database, STN: structural formula, SEQ ID NO: 1-48, siRNA, miRNA, RNAi, shRNA, 干扰RNA, RNA干扰, 寡聚核苷酸, 核苷酸类似物, 核酸类似物, 核苷类似物, 乙肝, small interfering RNA, RNA interfer+, small interference RNA, ODN, nucleotide, ribonucleotide, nucleotide analog, nucleotide analogue, HBV

**C. DOCUMENTS CONSIDERED TO BE RELEVANT**

| Category* | Citation of document, with indication, where appropriate, of the relevant passages | Relevant to claim No. |
|---|---|---|
| A | WO 2004084796 A2 (PHARMASSET LTD.) 07 October 2004 (2004-10-07)<br>entire document | 1-20 |
| A | WO 2018195165 A1 (ALNYLAM PHARMACEUTICALS, INC. et al.) 25 October 2018 (2018-10-25)<br>entire document | 1-20 |
| A | CN 108210510 A (SUZHOU RIBO LIFE SCIENCE CO., LTD.) 29 June 2018 (2018-06-29)<br>entire document | 1-20 |
| A | WO 2005020885 A2 (ISIS PHRAMACEUTICALS, INC.) 10 March 2005 (2005-03-10)<br>entire document | 1-20 |
| A | DRABIKOWSKA, A.K. et al. "Studies on the mechanism of antiviral action of 1-(β-D-Ribofuranosyl)-1,2,4-triazole-3-carboxamide(ribavirin)"<br>*Journal of Medicinal Chemistry,* Vol. 22, No. 6, 31 December 1979 (1979-12-31),<br>pp. 653-657 | 1-20 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **19 August 2021** | **09 September 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/098682**

**C. DOCUMENTS CONSIDERED TO BE RELEVANT**

| Category* | Citation of document, with indication, where appropriate, of the relevant passages | Relevant to claim No. |
|---|---|---|
| A | DUDYCZ, L. et al. "Synthesis and Determination of Antiviral Activity of the 2'(3')-O-Methyl Derivatives of Ribavirin(1-β-D-Ribofuranosyl-1,2,4-triazole-3-carboxamide)" *Journal of Medicinal Chemistry,* Vol. 20, No. 10, 31 December 1977 (1977-12-31), pp. 1354-1356 | 1-20 |
| A | NAIR, J. K. et al. "Multivalent N-Acetylgalactosamine-Conjugated siRNA localizes in Hepatocytes and Elicits Robust RNAi-Mediated Gene Silencing" *Journal of the American Chemical Society,* Vol. 136, No. 49, 01 December 2014 (2014-12-01), pp. 16958-16961 | 1-20 |

Form PCT/ISA/210 (second sheet) (January 2015)

**INTERNATIONAL SEARCH REPORT**

International application No.
**PCT/CN2021/098682**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2021/098682**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **20**
because they relate to subject matter not required to be searched by this Authority, namely:

[1] Claim 20 relates to a method for treating hepatitis B in a subject, and relates to a subject matter which the International Search Authority is not required to search under PCT Rule 39.1(iv): (4) a method for treatment of the human or animal body by surgery or therapy, and diagnostic methods. The international search is made on the basis of the method for preparing the corresponding drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/098682**

| Patent document cited in search report | Publication date (day/month/year) | Patent family member(s) | Publication date (day/month/year) |
|---|---|---|---|
| WO 2004084796 A2 | 07 October 2004 | BR PI0408846 A | 04 July 2006 |
| | | CN 1980678 A | 13 June 2007 |
| | | MX PA05010419 A | 31 May 2006 |
| | | US 2005049204 A1 | 03 March 2005 |
| | | RU 2005133093 A | 27 July 2006 |
| | | JP 2006524227 A | 26 October 2006 |
| | | CA 2529311 A1 | 07 October 2004 |
| | | AU 2004224575 A1 | 07 October 2004 |
| | | EP 1626692 A2 | 22 February 2006 |
| | | EP 1626692 A4 | 10 December 2008 |
| | | WO 2004084796 A3 | 06 April 2006 |
| WO 2018195165 A1 | 25 October 2018 | CN 110913898 A | 24 March 2020 |
| | | KR 20200015895 A | 13 February 2020 |
| | | BR 112019021852 A2 | 02 June 2020 |
| | | CA 3059446 A1 | 25 October 2018 |
| | | EP 3612219 A1 | 26 February 2020 |
| | | AU 2018254437 A1 | 28 November 2019 |
| | | US 2020038506 A1 | 06 February 2020 |
| | | TW 201906617 A | 16 February 2019 |
| | | PH 12019550220 A1 | 13 July 2020 |
| | | WO 2018195165 A8 | 22 November 2018 |
| | | SG 11201909572 Q A | 28 November 2019 |
| CN 108210510 A | 29 June 2018 | CN 108210510 B | 27 April 2021 |
| WO 2005020885 A2 | 10 March 2005 | WO 2005020885 A3 | 04 August 2005 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010529520
- CN 202011524835
- CN 202010524584
- CN 2020133982 W
- CN 202010522407
- CN 202011524307
- WO 2016077321 A
- WO 2018195165 A
- WO 2020036862 A
- WO 2015006740 A2


### Non-patent literature cited in the description


- **EDWARD J. G. et al.** The oral toll-like receptor-7 agonist GS-9620 in patients with chronic hepatitis B virus infection. *Journal of Hepatology,* 2015, vol. 63, 320-328
- **EDWARD J. G. et al.** The oral toll-like receptor 7 agonist GS-9620 in patients with chronic hepatitis B virus infection. *Journal of Hepatology,* 2015
- **ZHANG CHUNHONG.** Application of interferon in the treatment of hepatitis B. *Guide of China Medicine,* 2013, vol. 11, 475-476
- **JANSSEN et al.** *Lancet,* 2005, vol. 365, 123-129
- **MARCELLIN et al.** *N. Engl.J.Med.,* 2004, vol. 351, 1206-1217
- **BUSTER et al.** *Hepatology,* 2007, vol. 46, 388-394
- **JACKSON et al.** *RNA,* 2006, vol. 12, 1179-1187
- **IRIBE et al.** *ACS Omega,* 2017, vol. 2, 2055-2064
- **JANAS et al.** *Nat. Commun.,* 2018, vol. 9, 723-732
- **NAIR et al.** *Nucleic Acids Research,* 2017, vol. 45, 10969-10977